(19) Europäisches Patentamt
European Patent Office
Office européen des brevets

(11) **EP 2 833 130 A1**

(12) **EUROPEAN PATENT APPLICATION**

(43) Date of publication:
**04.02.2015 Bulletin 2015/06**

(51) Int Cl.:
**G01N 27/18** (2006.01)   **G01N 33/22** (2006.01)

(21) Application number: **14177437.2**

(22) Date of filing: **17.07.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **01.08.2013 JP 2013160732**

(71) Applicant: **Azbil Corporation**
**Chiyoda-ku**
**Tokyo 100-6419 (JP)**

(72) Inventor: **Ooishi, Yasuharu**
**Tokyo, Tokyo 100-6419 (JP)**

(74) Representative: **Tischner, Oliver**
**Lavoix Munich**
**Bayerstrasse 83**
**80335 München (DE)**

(54) **Calorific value calculation formula creation system, method of creating a calorific value calculation formula, calorific value measurement system, and method of measuring a calorific value**

(57)    A measurement module is configured to measure a value of an electric signal from a heater element in contact with mixed gas. A calculation formula creation module is configured to create a calorific value calculation formula based on calorific values of the plurality of kinds of mixed gas, on measured values of electric signals from the heater element, the heater element being in contact with each of the plurality of kinds of mixed gas having a first pressure, the heater element generating heat at a plurality of different temperatures, and on measured values of electric signals from the heater element, the heater element being in contact with each of the plurality of kinds of mixed gas having a second pressure different from the first pressure, where the values of electric signals from the heater element, the heater element generating heat at the plurality of different temperatures, are independent variables, and a calorific value of the mixed gas is a dependent variable.

FIG.2

**Description**

<u>Background</u>

The present invention relates to a calorific value calculation formula creation system, a method of creating a calorific value calculation formula, a calorific value measurement system, and a method of measuring a calorific value using a calorific value calculation formula capable of calculating a calorific value of mixed gas.

In the past, as this kind of calorific value calculation formula calculation system, there is known a system configured to create a calorific value calculation formula where heat loss coefficients or thermal conductivities at a plurality of different temperatures are independent variables and a calorific value is a dependent variable. The calorific value calculation formula is created based on known calorific values of a plurality of kinds of mixed gas, and based on heat loss coefficients or thermal conductivities measured at a plurality of different temperatures (for example, see International Publication No. 2010/038285).

<u>Summary</u>

According to International Publication No. 2010/038285, a calorific value calculation formula calculation system is configured to introduce measurement target mixed gas in a chamber, to measure heat loss coefficients or thermal conductivities of the mixed gas at a plurality of different temperatures, to substitute them in the independent variables of the calorific value calculation formula, and to thereby calculate a calorific value of mixed gas. However, because the pressure of a chamber may fluctuate largely, calculation accuracy of a calorific value, which is calculated based on the calorific value calculation formula created by the calorific value calculation formula creation system of International Publication No. 2010/038285, may be decreased when the pressure of mixed gas fluctuates.

In view of the above-mentioned circumstances, it is an object of the present invention to provide a calorific value calculation formula creation system, a method of creating a calorific value calculation formula, a calorific value measurement system, and a method of measuring a calorific value capable of preventing calculation accuracy from being decreased even if the pressure of mixed gas fluctuates.

According to the present invention, there is provided a calorific value calculation formula creation system, including: a heater element coming in contact with mixed gas; a measurement module configured to measure a value of an electric signal from the heater element; and a calculation formula creation module configured to create a calorific value calculation formula based on calorific values of the plurality of kinds of mixed gas, on measured values of electric signals from the heater element, the heater element being in contact with each of the plurality of kinds of mixed gas having a first pressure, the heater element generating heat at a plurality of different temperatures, and on measured values of electric signals from the heater element, the heater element being in contact with each of the plurality of kinds of mixed gas having a second pressure different from the first pressure, where the values of electric signals from the heater element, the heater element generating heat at the plurality of different temperatures, are independent variables, and a calorific value of the mixed gas is a dependent variable.

Further, according to the present invention, there is provided a method of creating a calorific value calculation formula, including: measuring a value of an electric signal from a heater element coming in contact with mixed gas; and creating a calorific value calculation formula based on calorific values of the plurality of kinds of mixed gas, on measured values of electric signals from the heater element, the heater element being in contact with each of the plurality of kinds of mixed gas having a first pressure, the heater element generating heat at a plurality of different temperatures, and on measured values of electric signals from the heater element, the heater element being in contact with each of the plurality of kinds of mixed gas having a second pressure different from the first pressure, where the values of electric signals from the heater element, the heater element generating heat at the plurality of different temperatures, are independent variables, and a calorific value of the mixed gas is a dependent variable.

Further, according to the present invention, there is provided a calorific value measurement system, including: a heater element coming in contact with mixed gas; a measurement module configured to measure a value of an electric signal from the heater element; and a calorific value calculation module configured to substitute measured values of electric signals from the heater element, the heater element generating heat at a plurality of different temperatures, in independent variables of a calorific value calculation formula, where the values of electric signals from the heater element, the heater element generating heat at the plurality of different temperatures, are the independent variables, and a calorific value of the mixed gas is a dependent variable, and to calculate the calorific value of the mixed gas. The calorific value calculation formula is created based on calorific values of the plurality of kinds of mixed gas, on measured values of electric signals from the heater element, the heater element being in contact with each of the plurality of kinds of mixed gas having a first pressure, the heater element generating heat at a plurality of different temperatures, and on measured values of electric signals from the heater element, the heater element being in contact with each of the plurality of kinds of mixed gas having a second pressure different from the first pressure.

Further, according to the present invention, there is provided a method of measuring a calorific value, including: measuring a value of an electric signal from a heater element coming in contact with mixed gas; and substituting measured values of electric signals from the heater element, the heater element generating heat at a plurality of different temperatures, in independent variables of a calorific value calculation formula, and calculating a calorific value of the mixed gas, where the values of electric signals from the heater element, the heater element generating heat at the plurality of different temperatures, are the independent variables, and a calorific value of the mixed gas is a dependent variable. The calorific value calculation formula is created based on calorific values of the plurality of kinds of mixed gas, on measured values of electric signals from the heater element, the heater element being in contact with each of the plurality of kinds of mixed gas having a first pressure, the heater element generating heat at a plurality of different temperatures, and on measured values of electric signals from the heater element, the heater element being in contact with each of the plurality of kinds of mixed gas having a second pressure different from the first pressure.

According to the calorific value calculation formula creation system, the method of creating a calorific value calculation formula, the calorific value measurement system, and the method of measuring a calorific value of the present invention, it is possible to prevent calculation accuracy of a calorific value calculated based on the calorific value calculation formula from being decreased even if the pressure of mixed gas fluctuates.

Brief Description of Drawings

Fig. 1 is a diagram schematically showing a configuration of the calorific value calculation formula creation system according to an embodiment;

Fig. 2 is a perspective view showing a microchip of Fig. 1;

Fig. 3 is a cross-sectional view illustrating the microchip taken along the line II-II of Fig. 2;

Fig. 4 is a circuit diagram showing an example of the circuit including a heater element of Fig. 2 and Fig. 3;

Fig. 5 is a circuit diagram showing an example of a circuit including a first temperature measurement element of Fig. 2 and Fig. 3;

Fig. 6 is a circuit diagram showing an example of the circuit including a heat-retaining element of Fig. 2 and Fig. 3;

Fig. 7 is a graph showing an example of the relationship between temperature of the heater element of Fig. 2 and Fig. 3 and heat loss coefficients of gas;

Fig. 8 is a diagram schematically showing the configuration of a calorific value calculation formula creation system including the calorific value calculation formula creation system of Fig. 1;

Fig. 9 is a flowchart illustrating an example of the behavior of the calorific value calculation formula creation system of Fig. 1 when the calorific value calculation formula creation system creates a calorific value calculation formula of mixed gas;

Fig. 10 is a diagram schematically showing the configuration of the calorific value measurement system of another embodiment;

Fig. 11 is a flowchart illustrating an example of the behavior of the calorific value measurement system of Fig. 10 when the calorific value measurement system measures a calorific value of mixed gas;

Fig. 12 is a graph showing an example of relationship between pressure of mixed gas and an error (i.e., difference between calculated calorific value and true value) of a calorific value calculated based on the calorific value calculation formula of the present invention;

Fig. 13 is a graph showing an example of relationship between pressure of mixed gas and an error (i.e., difference between calculated calorific value and true value) of a calorific value calculated based on the calorific value calculation formula of the present invention;

Fig. 14 is a graph showing an example of relationship between pressure of mixed gas and an error (i.e., difference between calculated calorific value and true value) of a calorific value calculated based on the calorific value calculation formula of the present invention;

Fig. 15 is a graph showing an example of relationship between pressure of mixed gas and an error (i.e., difference between calculated calorific value and true value) of a calorific value calculated based on the calorific value calculation formula of the present invention;

Fig. 16 is a graph showing an example of relationship between pressure of mixed gas and an error (i.e., difference between calculated calorific value and true value) of a calorific value calculated based on the calorific value calculation formula of the present invention; and

Fig. 17 is a graph showing another example of relationship between pressure of mixed gas and an error (i.e., difference between calculated calorific value and true value) of a calorific value calculated based on the calorific value calculation formula of the present invention.

Deticant Description of Embodiments

Detailed Description of Embodiments

Hereinafter, embodiments of the present invention will be described. The same or similar components are denoted by the same or similar reference symbols in the attached drawings. Note that because the drawings are schematically illustrated, for example, specific dimensions need to be determined in the light of the following description. In addition, as a matter of course, the same components may have different dimensions or ratios in the drawings. Note that, in the following description, the upper side, the lower side, the left side, and the right side of the sheets will be referred to as "upper", "bottom", "left", and "right", respectively.

(Calorific value calculation formula creation system and method of creating a calorific value calculation formula)

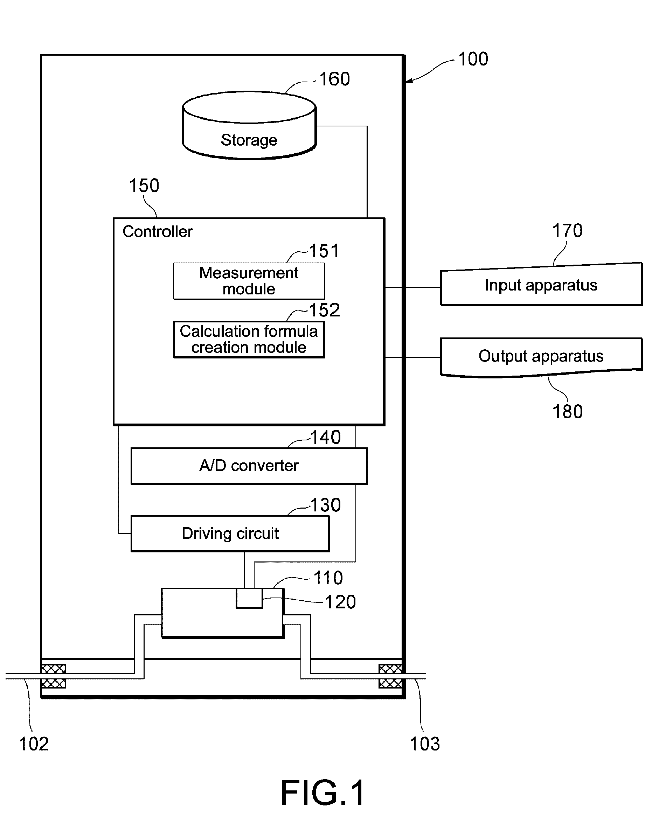

Fig. 1 to Fig. 9 illustrate a calorific value calculation formula creation system and a method of creating a calorific value calculation formula according to an embodiment of the present invention. Fig. 1 is a diagram schematically showing the configuration of the calorific value calculation formula creation system 100 of this embodiment. As shown in Fig. 1, the calorific value calculation formula creation system 100 is configured to create a calorific value calculation formula for calculating a calorific value of mixed gas including a plurality of kinds of gas components, e.g., gas-phase liquified natural gas, etc. The calorific value calculation formula creation system 100 includes the chamber 110, the microchip 120, the driving circuit 130, the A/D converter 140, the controller 150, the storage 160, the input apparatus 170, and the output apparatus 180.

The chamber 110 is a container in which mixed gas is introduced. The flow path 102 and the flow path 103 are connected to the chamber 101. The flow path 102 transfers mixed gas to the chamber 101. The flow path 103 exhausts the mixed gas from the chamber 101 to the outside. Further, the microchip 120 is arranged in the chamber 110 with the insulating member 18 (described later) interposed therebetween.

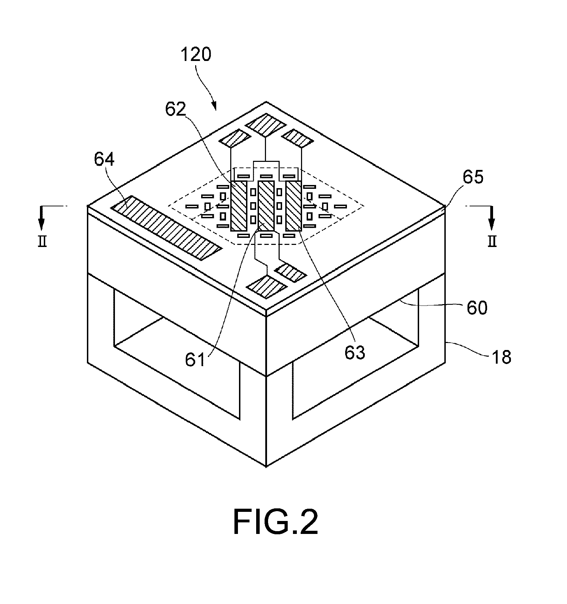

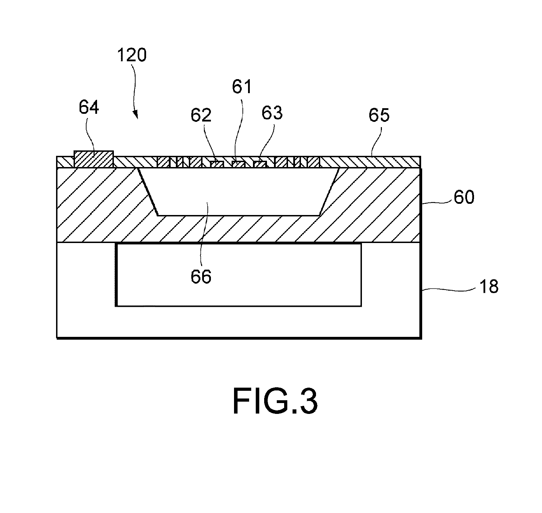

Fig. 2 is a perspective view showing the microchip 120 of Fig. 1. Fig. 3 is a cross-sectional view illustrating the microchip taken along the line II-II of Fig. 2. As shown in Fig. 2 and Fig. 3, the microchip 120 includes the substrate 60, the heater element 61, the first temperature measurement element 62, the second temperature measurement element 63, the heat-retaining element 64, and the insulating film 65.

The substrate 60 has the cavity 66. The cavity 66 is an opening at one surface (upper surface of each of Fig. 2 and Fig. 3) of the substrate 60. The thickness of the substrate 60 is, for example, 0.5 [mm]. The substrate 60 is about 1.5 [mm] square, for example. The insulating film 65 is arranged on the substrate 60 such that the insulating film 65 covers the cavity 66 of the substrate 60. The portion of the insulating film 65 covering the cavity 66 functions as a heat insulating diaphragm. The heater element 61, the first temperature measurement element 62, and the second temperature measurement element 63 are provided on the diaphragm portion of the insulating film 65. The heater element 61 is provided between the first temperature measurement element 62 and the second temperature measurement element 63. Further the heat-retaining element 64 is provided on the substrate 60.

The heater element 61 is arranged at the center of the diaphragm portion of the insulating film 65 covering the cavity 66. The heater element 61 is, for example, a resistor and is supplied with power to generate heat, to thereby heat gas in contact with the heater element 61. The first temperature measurement element 62 and the second temperature measurement element 63 are, for example, passive elements such as resistors, and output electric signals depending on the gas temperature. Hereinafter, an example in which an output signal from the first temperature measurement element 62 is used will be described, but the present invention is not limited thereto. For example, an output signal from the second temperature measurement element 63 may be used. Alternatively, an average value of an output signal from the first temperature measurement element 62 and an output signal from the second temperature measurement element 63 may be used as the output signal from the temperature measurement elements.

The heat-retaining element 64 is, for example, a resistor and is supplied with power to generate heat, to thereby keep the temperature of the substrate 60 constant. The substrate 60 may be made from silicon (Si) or the like. The insulating film 65 may be made from silicon oxide ($SiO_2$) or the like. The cavity 66 is formed by anisotropic etching or the like. In addition, the heater element 61, the first temperature measurement element 62, the second temperature measurement element 63, and the heat-retaining element 64 may be made from platinum (Pt) and may be formed by using a lithography method or the like. In addition, the heater element 61, the first temperature measurement element 62, and the second temperature measurement element 63 may be formed of the same members.

The microchip 120 is fixed on a container such as a chamber, which is to be filled up with gas, with a heat insulating member 18 provided on the bottom of the microchip 120, interposed therebetween. When the microchip 120 is fixed on, for example, the chamber with the heat insulating member 18 interposed therebetween, the temperature of the microchip 120 is less likely to be affected by variation in temperature of the inner wall and the like of the chamber. For example, the thermal conductivity of the heat insulating member 18 made of glass or the like is equal to or less than 1.0 [W/(m•K)].

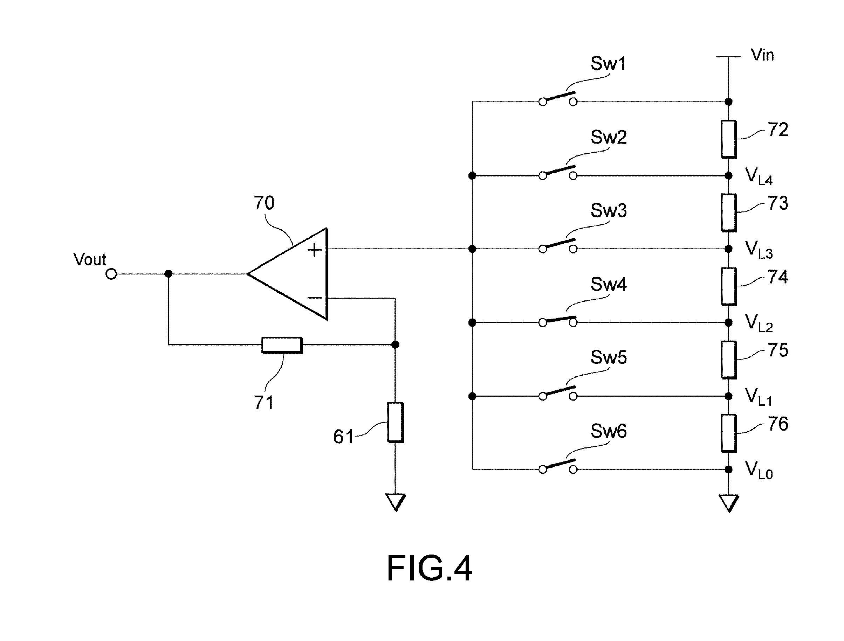

Fig. 4 is a circuit diagram showing an example of the circuit including the heater element 61 of Fig. 2 and Fig.

3. As shown in Fig. 4, the heater element 61 has one end electrically connected to, for example, an inverting input terminal (negative (-) input terminal) of the operational amplifier 70, and the other end connected to the ground. Further, the resistive element 71 is connected in parallel to the operational amplifier 70 between the inverting input terminal and the output terminal thereof. The non-inverting input terminal (positive (+) input terminal) of the operational amplifier 70 is electrically connected to a power source, a point between the resistive element 72 and the resistive element 73 connected in series to each other, a point between the resistive element 73 and the resistive element 74 connected in series to each other, a point between the resistive element 74 and the resistive element 75 connected in series to each other, a point between the resistive element 75 and the resistive element 76 connected in series to each other, or the ground terminal of the resistive element 76, selectively. The resistance values of the resistive elements 72, 73, 74, 75, and 76 are appropriately determined. As a result, when a voltage $V_{in}$ is applied to one end of the resistive element 72, a voltage $V_{L4}$, for example, is generated between the resistive element 73 and the resistive element 72. Similarly, a voltage $V_{L3}$, for example, is generated between the resistive element 74 and the resistive element 73, a voltage $V_{L2}$, for example, is generated between the resistive element 75 and the resistive element 74, and a voltage $V_{L1}$, for example, is generated between the resistive element 76 and the resistive element 75.

A switch Sw1 is provided between the power source and the non-inverting input terminal of the operational amplifier 70, a switch Sw2 is provided between the non-inverting input terminal of the operational amplifier 70 and the point between the resistive element 72 and the resistive element 73, and a switch Sw3 is provided between the non-inverting input terminal of the operational amplifier 70 and the point between the resistive element 73 and the resistive element 74. In addition, a switch Sw4 is provided between the non-inverting input terminal of the operational amplifier 70 and the point between the resistive element 74 and the resistive element 75, a switch Sw5 is provided between the non-inverting input terminal of the operational amplifier 70 and the point between the resistive element 75 and the resistive element 76, and a switch Sw6 is provided between the ground terminal of the resistive element 76 and the non-inverting input terminal of the operational amplifier 70.

If a voltage $V_{in}$ is applied to the non-inverting input terminal of the operational amplifier 70, only the switch Sw1 is turned on (closed) and energized, and the switches Sw2, Sw3, Sw4, Sw5, and Sw6 are turned off (opened) and disconnected. If a voltage $V_{L4}$ is applied to the non-inverting input terminal of the operational amplifier 70, only the switch Sw2 is turned on (closed) and energized, and the switches Sw1, Sw3, Sw4, Sw5, and Sw6 are turned off (opened) and disconnected. If a voltage $V_{L3}$ is applied to the non-inverting input terminal of the operational amplifier 70, only the switch Sw3 is turned on (closed) and energized, and the switches Sw1, Sw2, Sw4, Sw5, and Sw6 are turned off (opened) and disconnected. If a voltage $V_{L2}$ is applied to the non-inverting input terminal of the operational amplifier 70, only the switch Sw4 is turned on (closed) and energized, and the switches Sw1, Sw2, Sw3, Sw5, and Sw6 are turned off (opened) and disconnected. If a voltage $V_{L1}$ is applied to the non-inverting input terminal of the operational amplifier 70, only the switch Sw5 is turned on (closed) and energized, and the switches Sw1, Sw2, Sw3, Sw4, and Sw6 are turned off (opened) and disconnected. If a voltage $V_{L0}$ is applied to the non-inverting input terminal of the operational amplifier 70, only the switch Sw6 is turned on (closed) and energized, and the switches Sw1, Sw2, Sw3, Sw4, and Sw5 are turned off (opened) and disconnected. Therefore, 0 [V] or a voltage of one of five levels may be applied to the non-inverting input terminal of the operational amplifier 70 by turning on and off (closing and opening) the switches Sw1, Sw2, Sw3, Sw4, Sw5, and Sw6. That is, an application voltage which determines the temperature of the heater element 61 can be set in five levels by turning on and off (closing and opening) the switches Sw1, Sw2, Sw3, Sw4, Sw5, and Sw6.

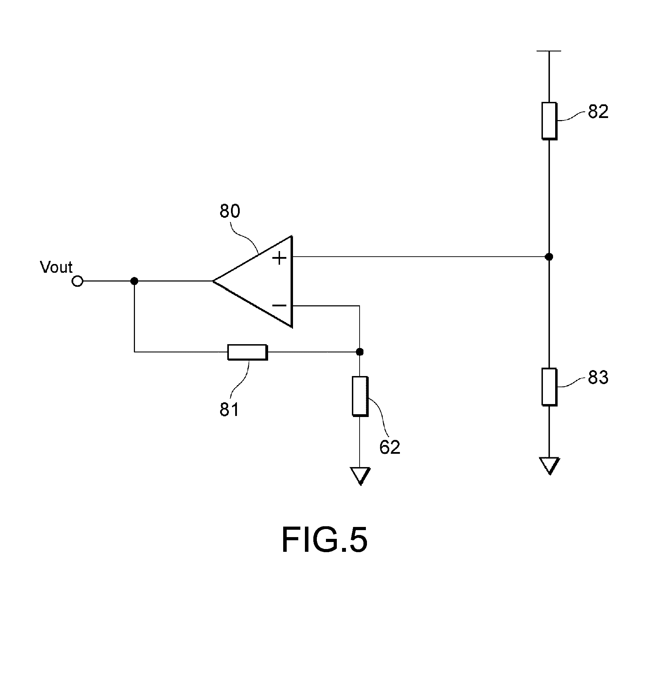

Fig. 5 is a circuit diagram showing an example of the circuit including the first temperature measurement element 62 of Fig. 2 and Fig. 3. As shown in Fig. 5, the first temperature measurement element 62 is electrically connected to, for example, the inverting input terminal (negative (-) input terminal) of the operational amplifier 80 at one end, and is connected to the ground at the other end. In addition, the resistive element 81 is connected in parallel to the operational amplifier 80 between the inverting input terminal and the output terminal thereof. The non-inverting input terminal (positive (+) input terminal) of the operational amplifier 80 is electrically connected to a point between the resistive element 82 and the resistive element 83, which are connected in series. The resistance values of the resistive elements 81, 82, and 83 are determined appropriately. A weak voltage of for example 0.3 [V], which is not enough to generate self-heating, is thus applied to the first temperature measurement element 62.

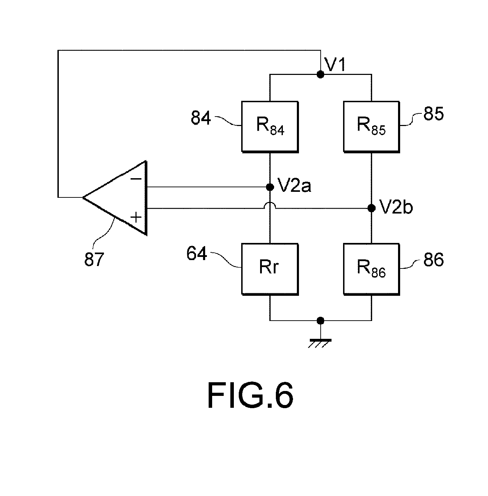

Fig. 6 is a circuit diagram showing an example of the circuit including the heat-retaining element 64 of Fig. 2 and Fig. 3. As shown in Fig. 6, a resistance bridge circuit includes the heat-retaining element 64. The resistance bridge circuit further includes the resistive element 84, the resistive element 85, and the resistive element 86. The resistive element 84 is connected in series to the heat-retaining element 64. The resistive element 85 and the resistive element 86 are connected in parallel to the heat-retaining element 64 and the resistive element 84. Here, the resistance value of the heat-retaining element 64 is Rr. The fixed resistance values of the resistive elements 84, 85, and 86 are $R_{84}$, $R_{85}$, and $R_{86}$, respectively. The operational amplifier 87 is connected to the resistance bridge circuit. The bridge driving voltage $V_1$ is feedback-controlled such that the bridge voltage $V_{2a}$ between the resistive element 84 and the heat-retaining element 64 is the same as the bridge voltage $V_{2b}$ between the resistive element 85 and the resistive element 86. As a result, the resistance value $R_r$ of the heat-retaining element 64 becomes constant, whereby the heat-retaining element 64 generates heat at a constant temperature.

The driving circuit 130 of Fig. 1 is configured to supply driving power, and is connected to the microchip 120 and the controller 150. The driving circuit 130 turns off and on (opens and closes) the switches Sw1, Sw2, Sw3, Sw4, Sw5, and Sw6 of Fig. 4 based on control signals input from the controller 150. As a result, the driving circuit 130 supplies driving power of a predetermined power (predetermined watt) to the heater element 61 of the microchip 120 of Fig. 2 and Fig. 3. When the driving circuit 130 supplies driving power to the heater element 61, the heater element 61 generates heat at a temperature corresponding to the power (watt) of the driving power. As a result, the heater element 61 is capable of generating heat at a plurality of different temperatures.

The A/D converter 140 of Fig. 1 is configured to convert analog electric signals (hereinafter arbitrarily referred to as input signals) to digital electric signals (hereinafter arbitrarily referred to as output signals), and is connected to the microchip 120 and the controller 150. The A/D converter 140 converts an input signal, which is input from the heater element 61 of the microchip 120, to an output signal, and outputs the output signal. Further, the A/D converter 140 converts an input signal, which is input from the first temperature measurement element 62 of the microchip 120, to an output signal, and outputs the output signal. For example, if the A/D converter 140 is a double integrator, the A/D converter 140 outputs a count value as an output signal. Note that the A/D converter 140 may process (e.g., filtering, signal amplification, etc.) an input signal, convert the processed signal to an output signal, and output the output signal.

The controller 150 of Fig. 1 is configured to control the respective blocks of the calorific value calculation formula creation system 100. Further, the controller 150 is configured to execute a method of creating a calorific value calculation the formula (described later) according to the present invention. The controller 150 includes (i.e., functions as) the measurement module 151 and the calculation formula creation module 152.

The measurement module 151 is configured to measure a value of an electric signal from the heater element 61, which is in contact with mixed gas in the chamber 110. Further, the measurement module 151 may measure a value of an electric signal from the first temperature measurement element 62 in contact with the mixed gas.

The calculation formula creation module 152 is configured to create a calorific value calculation formula for calculating a calorific value of measurement target mixed gas, whose calorific value is unknown.

Note that the controller 150 includes, for example, a CPU (Central Processing Unit), a GPU (Graphic Processing Unit), memories such as a ROM (Read Only Memory), a RAM (Random Access Memory), and a buffer, various interfaces such as an input interface and an output interface, and a bus connecting them. Meanwhile, a computer (microprocessor) may execute a program to thereby realize functions of the respective blocks of the controller 150. The respective blocks of the controller 150 may thus be realized by hardware, software, or the combination of hardware and software. The present technology is not limited to any one of them.

The storage 160 of Fig. 1 is configured to store data. The controller 150 is connected to the storage 160 such that the controller 150 is capable of accessing the storage 160. The storage 160 is configured to store, for example, measured values of electric signals from the heater element 61 and the first temperature measurement element 62 measured by the measurement module 151, a calorific value calculation formula created by the calculation formula creation module 152, and the like.

A user inputs information by using the input apparatus 170 of Fig. 1. The input apparatus 170 is, for example, a keyboard, a pointing device such as a mouse, or the like. The input apparatus 170 is connected to the controller 150. The input apparatus 170 inputs various kinds of information in the controller 150.

The output apparatus 180 of Fig. 1 is configured to output information for a user. The output apparatus 180 may be an image display apparatus such as a liquid crystal display or a monitor, a printer, or the like. The output apparatus 180 is connected to the controller 150, and outputs information input from the controller 150.

Next, a calorific value calculation formula of mixed gas will be described.

The resistance value of the heater element 61 shown in Figs. 1 and 2 varies depending on the temperature of the heater element 61. The relationship between the temperature $T_H$ of the heater element 61 and the resistance $R_H$ of the heater element 61 is represented by the following Expression (1):

$$R_H = R_{H\_STD} \times [1 + \alpha_H(T_H - T_{H\_STD}) + \beta_H(T_H - T_{H\_STD})^2] \,...(1)$$

where the symbol $T_{H\_STD}$ represents the standard temperature of the heater element 61 and is, for example, 20°C. The symbol $R_{H\_STD}$ represents the resistance value of the heater element 61 which is measured at the standard temperature $T_{H\_STD}$ in advance. The symbol $\alpha_H$ represents a primary resistance-temperature coefficient. The symbol $\beta_H$ represents a secondary resistance-temperature coefficient.

The resistance value $R_H$ of the heater element 61 is calculated from the driving power $P_H$ of the heater element 61 and the on-current IH of the heater element 61 by the following Expression (2):

$$R_H = P_H/I_H{}^2 \ ...(2)$$

Alternatively, the resistance value $R_H$ of the heater element 61 is calculated from a voltage $V_H$ applied to the heater element 61 and the on-current $I_H$ of the heater element 61 by the following Expression (3):

$$R_H = V_H/I_H \ ...(3)$$

Here, the temperature $T_H$ of the heater element 61 is stabilized when the heater element 61 and the atmospheric gas are thermally balanced. The thermally balanced state is a state in which the heat generation of the heater element 61 and the heat loss to the atmospheric gas from the heater element 61 are balanced. As shown in the following Expression (4), by dividing driving power $P_H$ of the heater element 61 in the balanced state by a difference $\Delta T_H$ between the temperature TH of the heater element 61 and the temperature $T_I$ of the atmospheric gas, a radiation coefficient $M_I$ of the atmospheric gas is obtained. The unit of the radiation coefficient $M_I$ is, for example, W/°C.

$$M_I = P_H/(T_H - T_I)$$
$$= P_H/\Delta T_H = (V_H{}^2/R_H)/\Delta T_H \ ...(4)$$

From the above Expression (1), the temperature $T_H$ of the heater element 61 is represented by the following Expression (5):

$$T_H = (1/2\beta_H) \times [-\alpha_H + [\alpha_H{}^2 - 4\beta_H(1 - R_H/R_{H\_STD})]^{1/2}] + T_{H\_STD} \ ...(5)$$

Accordingly, the difference $\Delta T_H$ between the temperature $T_H$ of the heater element 61 and the temperature $T_I$ of the atmospheric gas is represented by the following Expression (6):

$$\Delta T_H = (1/2\beta_H) \times [-\alpha_H + [\alpha_H{}^2 - 4\beta_H(1 - R_H/R_{H\_STD})]^{1/2}] + T_{H\_STD}{}^{-I} \ ...(6)$$

The temperature $T_I$ of the atmospheric gas is close to the temperature $T_I$ of the first temperature measurement element 62 which is supplied with power enough not to generate self-eating. The relationship between the temperature $T_I$ of the first temperature measurement element 62 and the resistance $R_I$ of the first temperature measurement element 62 is represented by the following Expression (7):

$$R_I = R_{I\_STD} \times [1 + \alpha_I(T_I - T_{I\_STD}) + \beta_I(T_I - T_{I\_STD})^2] \ ...(7)$$

where the symbol $T_{H\_STD}$ represents the standard temperature of the first temperature measurement element 62 and is, for example, 20°C. The symbol $R_{\_STD}$ represents the resistance value of the first temperature measurement element 62 which is measured at the standard temperature $T_{I\_STD}$ in advance. The symbol $\alpha_I$ represents a primary resistance-temperature coefficient. The symbol $\beta_I$ represents a secondary resistance-temperature coefficient.

From the above Expression (7), the temperature $T_I$ of the first temperature measurement element 62 is represented by the following Expression (8):

$$T_I = (1/2\beta_I) \times [-\alpha_I + [\alpha_I{}^2 - 4\beta_I(1 - R_I/R_{I\_STD})]^{1/2}] + T_{I\_STD} \ ...(8)$$

As a result, the heat loss coefficient $M_I$ of the atmosphere gas is given by the formula (9) below.

$$M_I = P_H / \Delta T_H$$

$$= P_H / [(1/2\beta_H)[-\alpha_H + [\alpha_H{}^2 - 4\beta_H(1 - R_H/R_{H\_STD})]^{1/2}] + T_{H\_STD} - (1/2\beta_I)[-\alpha_I + [\alpha_I{}^2 - 4\beta_I(1 - R_I/R_{I\_STD})]^{1/2}] - T_{I\_STD}] \ldots (9)$$

Since the line current $I_H$, driving power $P_H$, or voltage $V_H$ of the heater element 61 can be measured, the resistance value $R_H$ of the heater element 61 can be calculated from the above formula (2) or (3). Likewise, the resistance value $R_I$ of the first temperature measurement element 62 can also be calculated. Therefore, the heat loss coefficient $M_I$ of the atmosphere gas can be calculated from the formula (9) using the microchip 8.

Since the heat-retaining element 64 maintains the temperature of the substrate 60 to be constant, the temperature of the atmosphere gas in the vicinity of the microchip 8 before the heater element 61 generates heat is approximate to the constant temperature of the substrate 60. Therefore, a variation in the temperature of the atmosphere gas before the heater element 61 generates heat is prevented. Since the heater element 61 heats the atmosphere gas whose temperature variation has been prevented, it is possible to calculate the radiation coefficient $M_I$ with high accuracy.

It is assumed that the atmosphere gas is a mixed gas and the mixed gas includes four kinds of gas components A, B, C, and D. The sum of the volume ratio $V_A$ of the gas A, the volume ratio $V_B$ of the gas B, the volume ratio $V_C$ of the gas C, and the volume ratio $V_D$ of the gas D is 1, as represented by the following Expression (10):

$$V_A + V_B + V_C + V_D = 1 \ldots (10)$$

When the calorific value of the gas A per unit volume is $K_A$, the calorific value of the gas B per unit volume is $K_B$, the calorific value of the gas C per unit volume is $K_C$, and the calorific value of the gas D per unit volume is $K_D$, the calorific value Q of the mixed gas per unit volume is the sum of the values obtained by multiplying the volume ratios of the gas components by the calorific values of the gas components per unit volume. Therefore, the calorific value Q of the mixed gas per unit volume is represented by the following Expression (11):

$$Q = K_A \times V_A + K_B \times V_B + K_C \times V_C + K_D \times V_D \ldots (11)$$

The unit of the calorific value per unit volume is, for example, $MJ/m^3$.

When the radiation coefficient of the gas A is $M_A$, the radiation coefficient of the gas B is $M_B$, the radiation coefficient of the gas C is $M_C$, and the radiation coefficient of the gas D is $M_D$, the radiation coefficient $M_I$ of the mixed gas is the sum of the values obtained by multiplying the volume ratios of the gas components by the radiation coefficients of the gas components. Therefore, the radiation coefficient $M_I$ of the mixed gas is represented by the following Expression (12):

$$M_I = M_A \times V_A + M_B \times V_B + M_C \times V_C + M_D \times V_D \ldots (12)$$

Further, since the radiation coefficient of gas depends on the heat generating temperature $T_H$ of the heater element 61, the radiation coefficient $M_I$ of the mixed gas is represented as a function of the temperature $T_H$ of the heater element 61 by the following Expression (13):

$$M_I = M_A \times V_A + M_B \times V_B + M_C \times V_C + M_D \times V_D \ldots (13)$$

As a result, the heat loss coefficient $M_{I1}(T_{H1})$ of the mixed gas is given by the formula (14) below when the temperature of the heater element 61 is $T_{H1}$. In addition, the heat loss coefficient $M_{I2}(T_{H2})$ of the mixed gas is given by the formula (15) below when the temperature of the heater element 61 is $T_{H2}$, and the heat loss coefficient $M_{I3}(T_{H3})$ of the mixed gas is given by the formula (16) below when the temperature of the heater element 61 is $T_{H3}$, where the temperatures $TH_1$, $TH_2$, and $T_{H3}$ of the heater element 61 are different from each other.

$$M_{I1}(T_{H1})=M_A(T_{H1})xV_A+M_B(T_{H1})xV_B+M_C(T_{H1})xV_C+M_D(T_{H1})xV_D \ ...(14)$$

$$M_{I2}(T_{H2})=M_A(T_{H2})xV_A+M_B(T_{H2})xV_B+M_C(T_{H2})xV_C+M_D(T_{H2})xV_D \ ...(15)$$

$$M_{I3}(T_{H3})=M_A(T_{H3})xV_A+M_B(T_{H3})xV_B+M_C(T_{H3})xV_C+M_D(T_{H3})xV_D \ ...(16)$$

Here, when the radiation coefficients $M_A$ $(T_H)$, $M_B$ $(T_H)$, $M_C$ $(T_H)$, and $M_D$ $(T_H)$ of the gas components relative to the temperature $T_H$ of the heater element 61 have nonlinearity, the above Expressions (14) to (16) have a linear independent relationship. In addition, even when the radiation coefficients $M_A$ $(T_H)$, $M_B$ $(T_H)$, $M_C$ $(T_H)$, and $M_D$ $(T_H)$ of the gas components relative to the temperature $T_H$ of the heater element 61 have linearity, when the change ratios of the radiation coefficients $M_A$ $(T_H)$, $M_B$ $(T_H)$, $M_C(T_H)$, and $M_D$ $(T_H)$ of the gas components relative to the temperature TH of the heater element 61 are different from each other, the above Expressions (14) to (16) have a linear independent relationship. Further, when Expressions (14) to (16) have a linear independent relationship, Expressions (10) and (14) to (16) have a linear independent relationship.

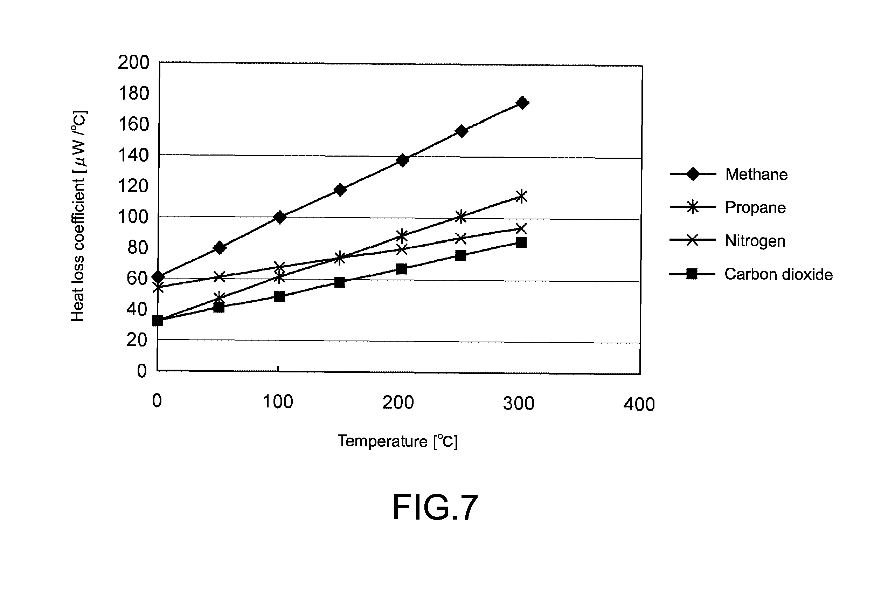

Fig. 7 is a graph showing an example of the relationship between temperature of the heater element 61 of Fig. 2 and Fig. 3 and heat loss coefficients of mixed gas. Note that the graph of Fig. 7 shows the heat loss coefficients of methane ($CH_4$), propane ($C_3H_8$), nitrogen ($N_2$), and carbon dioxide ($CO_2$), which are gas components of natural gas. As shown in Fig. 7, the heat loss coefficient of each gas component (methane ($CH_4$), propane ($C_3H_8$), nitrogen ($N_2$), or carbon dioxide ($CO_2$)) is linear with respect to the temperature of the heater element 61. Meanwhile, the change rate of the heat loss coefficient of each gas component (methane ($CH_4$), propane ($C_3H_8$), nitrogen ($N_2$), or carbon dioxide ($CO_2$)) to the temperature of the heater element 61 is different from each other. Because of this, if the gas components of mixed gas are methane ($CH_4$), propane ($C_3H_8$), nitrogen ($N_2$), and carbon dioxide ($CO_2$), each of the formula (14) to the formula (16) has a linear independent relationship.

Values of the heat loss coefficients $M_A$ $(T_{H1})$, $M_B$ $(T_{H1})$, $M_C$ $(T_{H1})$, $M_D$ $(T_{H1})$, $M_A$ $(T_{H2})$, $M_B$ $(T_{H2})$, $M_C$ $(T_{H2})$, $M_D$ $(T_{H2})$, $M_A$ $(T_{H3})$, $M_B$ $(T_{H3})$, $M_C$ $(T_{H3})$, and $M_D$ $(T_{H3})$ of the respective gas components in the formula (14) to (16) can be obtained in advance through measurement or the like. Therefore, if solving the simultaneous equations of the formula (10) and (14) to (16), each of the volume ratio $V_A$ of the gas A, the volume ratio $V_B$ of the gas B, the volume ratio $V_C$ of the gas C, and the volume ratio $V_D$ of the gas D is given as a function of the heat loss coefficients $M_{I1}$ $(T_{H1})$, $M_{I2}$ $(T_{H2})$, and $M_{I3}$ $(T_{H3})$ of the mixed gas as shown in the formula (17) to (20) below, where, in the formula (17)to the formula (20), n is a positive integer and $f_n$ represents a function.

$$V_A=f_1[M_{I1}(T_{H1}), M_{I2}(T_{H2}), M_{I3}(T_{H3})] \ ...(17)$$

$$V_B=f_2[M_{I1}(T_{H1}), M_{I2}(T_{H2}), M_{I3}(T_{H3})] \ ...(18)$$

$$V_C=f_3[M_{I1}(T_{H1}), M_{I2}(T_{H2}), M_{I3}(T_{H3})] \ ...(19)$$

$$V_D=f_4[M_{I1}(T_{H1}), M_{I2}(T_{H2}), M_{I3}(T_{H3})] \ ...(20)$$

Here, the formula (21) below can be obtained by substituting the formula (17) to (20) into the formula (11).

$$Q=K_A \times V_A + K_B \times V_B + K_C \times V_C + K_D \times V_D$$
$$=K_A \times f_1[M_{I1}(T_{H1}), M_{I2}(T_{H2}), M_{I3}(T_{H3})]$$
$$+K_B \times f_2[M_{I1}(T_{H1}), M_{I2}(T_{H2}), M_{I3}(T_{H3})]$$
$$+K_C \times f_3[M_{I1}(T_{H1}), M_{I2}(T_{H2}), M_{I3}(T_{H3})]$$
$$+K_D \times f_4[M_{I1}(T_{H1}), M_{I2}(T_{H2}), M_{I3}(T_{H3})] \dots(21)$$

As shown in the formula (21), the calorific value Q per unit volume of the mixed gas is given by a formula including the heat loss coefficients $M_{I1}(T_{H1})$, $M_{I2}(T_{H2})$, and $M_{I3}(T_{H3})$ of the mixed gas when the temperatures of the heater element 61 are $TH_1$, $T_{H2}$, and $T_{H3}$ as variables. As a result, the calorific value Q of the mixed gas is given by the formula (22) below, where g represents a function.

$$Q=g[M_{I1}(T_{H1}), M_{I2}(T_{H2}), M_{I3}(T_{H3})] \dots(22)$$

As a result, if the above formula (22) is obtained in advance for the mixed gas made up of the gas A, the gas B, the gas C, and the gas D, the calorific value Q per unit volume of the inspection subject mixed gas of which the volume ratio $V_A$ of the gas A, the volume ratio $V_B$ of the gas B, the volume ratio $V_C$ of the gas C, and the volume ratio $V_D$ of the gas D are unknown can be easily calculated.

In detail, by measuring the heat loss coefficients $M_{I1}(T_{H1})$, $M_{I2}(T_{H2})$, and $M_{I3}(T_{H3})$ of the inspection subject mixed gas when the temperatures of the heater element 61 are $TH_1$, $T_{H2}$, and $T_{H3}$ and then substituting them into the formula (22), it becomes possible to obtain a unique calorific value Q of the inspection subject mixed gas.

In addition, the heat loss coefficient $M_I$ of the mixed gas is dependent on the resistance value $R_H$ of the heater element 61 and the resistance value $R_I$ of the first temperature measurement element 62 as shown in the formula (9). Therefore, the inventors of the invention found that, as shown in the formula (23) below, the calorific value Q per unit volume of the mixed gas is also given by a formula including the resistance values $R_{H1}(T_{H1})$, $R_{H2}(T_{H2})$, and $R_{H3}(T_{H3})$ of the heater element 61 when the temperatures of the heater element 61 are $T_{H1}$, $T_{H2}$, and $T_{H3}$, and the resistance value $R_I$ of the first temperature measurement element 62 that comes into contact with the mixed gas, as variables.

$$Q=g[R_{H1}(T_{H1}), R_{H2}(T_{H2}), R_{H3}(T_{H3}), R_I] \dots(23)$$

Therefore, also by measuring the resistance values $R_{H1}(T_{H1})$, $R_{H2}(T_{H2})$, and $R_{H3}(T_{H3})$ of the heater element 61 that comes into the inspection subject mixed gas when the temperatures of the heater element 61 are $T_{H1}$, $T_{H2}$ and $T_{H3}$, and the resistance value $R_I$ of the first temperature measurement element 62 that comes into contact with the inspection subject mixed gas and then substituting them into the formula (23), it becomes possible to obtain a unique calorific value Q of the inspection subject mixed gas.

In addition, as shown in the formula (24) below, the calorific value Q per unit volume of the mixed gas is also given by a formula including the line currents $I_{H1}(T_{H1})$, $I_{H2}(T_{H2})$, and $I_{H3}(T_{H3})$ of the heater element 61 when the temperatures of the heater element 61 are $T_{H1}$, $T_{H2}$, and $T_{H3}$, and the line current $I_I$ of the first temperature measurement element 62 that comes into contact with the mixed gas as variables.

$$Q=g[I_{H1}(T_{H1}), I_{H2}(T_{H2}), I_{H3}(T_{H3}), I_I] \dots(24)$$

Alternately, as shown in the formula (25) below, the calorific value Q per unit volume of the mixed gas is also given by a formula including the voltages $V_{H1}(T_{H1})$, $V_{H2}(T_{H2})$, and $V_{H3}(T_{H3})$ applied to the heater element 61 when the temperatures of the heater element 61 are $T_{H1}$, $T_{H2}$, and $T_{H3}$, and the voltage $V_I$ applied to the first temperature measurement element 62 that comes into contact with the mixed gas, as variables.

$$Q=g[V_{H1}(T_{H1}),\ V_{H2}(T_{H2}),\ V_{H3}(T_{H3}),\ V_1]\ ...(25)$$

In addition, alternatively, as shown in the formula (26) below, the calorific value Q per unit volume of the mixed gas is also given by a formula including the output signals $AD_{H1}(T_{H1})$, $AD_{H2}(T_{H2})$, and $AD_{H3}(T_{H3})$ of the analog-digital converter (hereinafter referred to as "A/D converter"), which is connected to the heater element 61, when the temperatures of the heater element 61 are $T_{HI}$, $T_{H2}$, and $T_{H3}$, and the output signal $AD_I$ of the A/D converter, which is connected to the first temperature measurement element 62 that comes into contact with the mixed gas, as variables.

$$Q=g[AD_{I1}(T_{H1}),\ AD_{I2}(T_{H2}),\ AD_{I3}(T_{H3}),\ AD_1]\ ...(26)$$

As a result, as shown in the formula (27) below, the calorific value Q per unit volume of the mixed gas is also given by a formula including the electric signals $S_{H1}(T_{H1})$, $S_{H2}(T_{H2})$, and $S_{H3}(T_{H3})$ from the heater element 61 when the temperatures of the heater element 61 are $T_{H1}$, $T_{H2}$, and $T_{H3}$, and the electric signal $S_I$ from the first temperature measurement element 62 that comes into contact with the mixed gas, as variables.

$$Q=g[S_{H1}(T_{H1}),\ S_{H2}(T_{H2}),\ S_{H3}(T_{H3}),\ S_1]\ ...(27)$$

Further, if the temperature of mixed gas is constant, the electric signal $S_I$ from the first temperature measurement element 62 is a constant number. In this case, as shown in the following formula (28), the calorific value Q per unit volume of mixed gas is also obtained by a formula where only the electric signals $S_{H1}(T_{H1})$, $S_{H2}(T_{H2})$, and $S_{H3}(T_{H3})$ from the heater element 61 are variables.

$$Q = g[S_{H1}(T_{H1}),\ S_{H2}(T_{H2}),\ S_{H3}(T_{H3})]...(28)$$

Meanwhile, the gas components of the mixed gas are not limited to 4 kinds. For example, when the mixed gas is made up of n kinds of gas components, firstly, as shown in the formula (29), a formula including the electric signals $S_{H1}(T_{H1})$, $S_{H2}(T_{H2})$, $S_{H3}(T_{H3})$,..., $S_{Hn-1}(TH_{n-1})$ from the heater element 61 at the heating temperatures $TH_1$, $T_{H2}$, $T_{H3}$, ..., $T_{Hn-1}$ of at least n-1 kinds, and the electric signal SI from the first temperature measurement element 62 that comes into contact with the mixed gas, which are given in the formula (28) below, as variables, is obtained in advance. Then, the values of the electric signals $S_{H1}(T_{H1})$, $S_{H2}(T_{H2})$, $S_{H3}(T_{H3})$, ..., $S_{Hn-1}(TH_{n-1})$ from the heater element 61 that comes into contact with the inspection subject mixed gas of which each of the volume ratios of n kinds of gas components are unknown at the heating temperatures $T_{H1}$, $T_{H2}$, $T_{H3}$, ..., $T_{Hn-1}$ of n-1 kinds, and the value of the electric signal SI from the first temperature measurement element 62 that comes into contact with the inspection subject mixed gas, are measured and substituted into the formula (29), whereby it becomes possible to obtain the unique calorific value Q per unit volume of the inspection subject mixed gas.

$$Q=g[S_{H1}(T_{H1}),\ SD_{H2}(T_{H2}),\ SD_{H3}(T_{H3}),\ ...,\ S_{Hn-1}(T_{Hn-1}),\ S_1]\ ...(29)$$

However, if the mixed gas includes alkane ($C_jH_{2j+2}$), in which j is a natural number, other than methane ($CH_4$) and propane ($C_3H_8$) as the gas components in addition to methane ($CH_4$) and propane ($C_3H_8$), the calculation of the formula (28) is not affected even when alkane ($C_jH_{2j+2}$) other than methane ($CH_4$) and propane ($C_3H_8$) is considered as a mixture of methane ($CH_4$) and propane ($C_3H_8$). For example, it makes no change to consider, for example, ethane ($C_2H_6$), butane ($C_4H_{10}$), pentane ($C_5H_{12}$), and hexane ($C_6H_{14}$) as a mixture of methane ($CH_4$) and propane ($C_3H_8$) multiplied by a predetermined coefficient respectively and then calculate the formula (29) as shown in the formula (30) to (33).

$$C_2H_6 = 0.5\ CH_4 + 0.5\ C_3H_8 \quad \cdots \quad (30)$$

$$C_4H_{10} = -0.5\ CH_4 + 1.5\ C_3H_8 \quad \cdots \quad (31)$$

$$C_5H_{12} = -1.0\ CH_4 + 2.0\ C_3H_8 \quad \cdots \quad (32)$$

$$C_6H_{14} = -1.5\ CH_4 + 2.5\ C_3H_8 \quad \cdots \quad (33)$$

As a result, when the mixed gas made up of n kinds of gas components includes z kinds of alkane ($C_jH_{2j+2}$) other than methane ($CH_4$) and propane ($C_3H_8$) in addition to methane ($CH_4$) and propane ($C_3H_8$) as the gas components, in which z is a natural number, it is allowed to obtain a formula including the electric signals $S_H$ from the heater element 61 at a minimum of n-z-1 kinds of the heating temperatures and the electric signal $S_I$ from the first temperature measurement element 62 as variables.

When the kind of gas components in the mixed gas used to calculate Expression (29) is equal to the kind of gas components in a mixed gas to be examined whose calorific value Q per unit volume is unknown, Expression (29) may be used to calculate the calorific value Q of the mixed gas to be examined. When the mixed gas to be examined includes gas components that are smaller than n kinds of gas components and gas components smaller than n kinds of gas components are included in the mixed gas used to calculate Expression (29), Expression (29) can be used. For example, when the mixed gas used to calculate Expression (29) includes four kinds of gas components, that is, methane ($CH_4$), propane ($C_3H_8$), nitrogen ($N_2$), and carbon dioxide ($CO_2$) and the mixed gas to be examined does not include nitrogen ($N_2$), but includes only three kinds of gas components, that is, methane ($CH_4$), propane ($C_3H_8$), and carbon dioxide ($CO_2$), Expression (29) can also be used to calculate the calorific value Q of the mixed gas to be examined.

When the mixed gas used to calculate Expression (28) includes as gas components methane ($CH_4$) and propane ($C_3H_8$), Expression (28) can be used even when the mixed gas to be examined includes alkane ($C_jH_{2j+2}$) which is not included in the mixed gas used to calculate Expression (28). This is because regarding alkane ($C_jH_{2j+2}$) other than methane ($CH_4$) and propane ($C_3H_8$) as a mixture of methane ($CH_4$) and propane ($C_3H_8$) does not affect the calculation of the calorific value Q per unit volume by Expression (28), as described above.

Next, an example of the configuration of a system configured to create a calorific value calculation formula of mixed gas will be described.

Here, four kinds of mixed gas (hereinafter arbitrarily referred to as first to fourth sample mixed gases) having different known calorific values Q, respectively, are suppositionally prepared as samples in order to create a calorific value calculation formula of measurement target mixed gas. For example, the first to fourth sample mixed gases are gas-phase liquified natural gas having different known calorific values Q. Each of the first to fourth sample mixed gases includes, for example, four kinds of gas components, that is, methane ($CH_4$), propane ($C_3H_8$), ethane ($C_2H_6$), and butane ($C_4H_{10}$) at different rates (percentages).

Note that, in general, liquified natural gas may include pentane ($C_5H_{12}$) and hexane ($C_6H_{14}$) in addition to methane ($CH_4$), propane ($C_3H_8$), ethane ($C_2H_6$), and butane ($C_4H_{10}$). However, because liquified natural gas includes very little pentane ($C_5H_{12}$) and hexane ($C_6H_{14}$), the effect of pentane ($C_5H_{12}$) and hexane ($C_6H_{14}$) may be disregarded. Because of this, even if liquified natural gas includes at least one of pentane ($C_5H_{12}$) and hexane ($C_6H_{14}$), it may be considered that liquified natural gas is composed of four kinds of gas components, i.e., methane ($CH_4$), propane ($C_3H_8$), ethane ($C_2H_6$), and butane ($C_4H_{10}$).

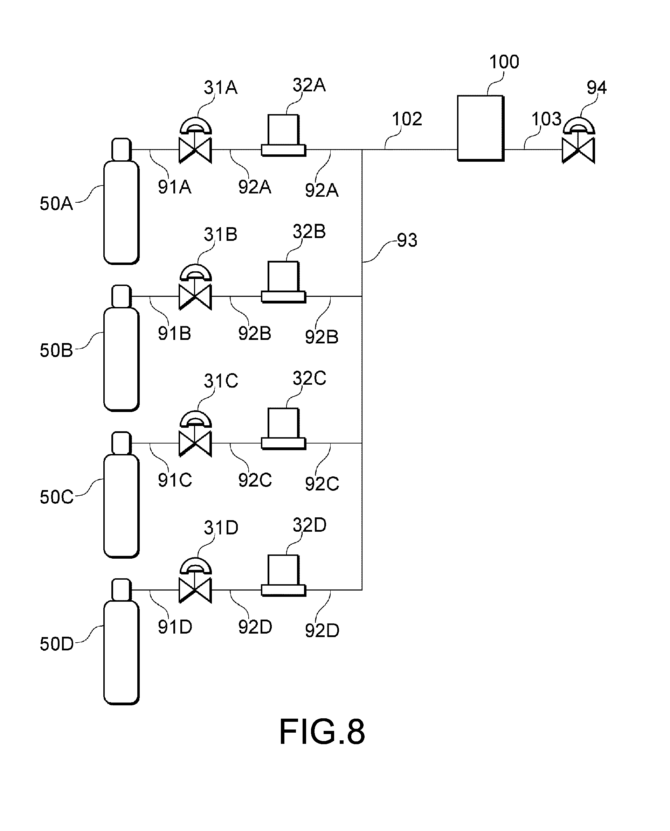

Fig. 8 is a diagram schematically showing the configuration of a system. The system includes the calorific value calculation formula creation system 100 of Fig. 1. As shown in Fig. 8, the first gas cylinder 50A storing a first sample mixed gas, the second gas cylinder 50B storing a second sample mixed gas, the third gas cylinder 50C storing a third sample mixed gas, and the fourth gas cylinder 50D storing a fourth sample mixed gas are prepared. The first gas pressure adjuster 31 A is configured to adjust the pressure of the first sample mixed gas, and is connected to the first gas cylinder 50A via the flow path 91 A. In addition, the first flow rate controller 32A is connected to the first gas pressure adjuster 31 A through the flow path 92A. The first flow rate controller 32A is configured to control the flow rate of the first sample mixed gas transferred to the calorific value calculation formula creation system 100 through the flow path 92A and a flow path 102.

The second gas pressure adjuster 31 B is configured to adjust the pressure of the second sample mixed gas, and is connected to the second gas cylinder 50B via the flow path 91 B. In addition, the second flow rate controller 32B is connected to the second gas pressure adjuster 31 B through the flow path 92B. The second flow rate controller 32B is configured to control the flow rate of the second sample mixed gas transferred to the calorific value calculation formula creation system 100 through the flow paths 92B, 93, and 102.

The third gas pressure adjuster 31 C is configured to adjust the pressure of the third sample mixed gas, and is connected to the third gas cylinder 50C via the flow path 91C. In addition, the third flow rate controller 32C is connected to the third gas pressure adjuster 31 C through the flow path 92C. The third flow rate controller 32C is configured to control the flow rate of the third sample mixed gas transferred to the calorific value calculation formula creation system 100 through the flow paths 92C, 93, and 102.

The fourth gas pressure adjuster 31 D is configured to adjust the pressure of the fourth sample mixed gas, and is connected to the fourth gas cylinder 50D via the flow path 91 D. In addition, the fourth flow rate controller 32D is connected to the fourth gas pressure adjuster 31 D through the flow path 92D. The fourth flow rate controller 32D is configured to control the flow rate of the fourth sample mixed gas transferred to the calorific value calculation formula creation system 100 through the flow paths 92D, 93, and 102.

Further, the gas the pressure adjuster 94 is connected to the flow path 103. The gas the pressure adjuster 94 is configured to adjust the pressure of the first to fourth sample mixed gases introduced in the chamber 110 of the calorific value calculation formula creation system 100 of Fig. 1.

Next, the behavior of the calorific value calculation formula creation system 100 when the calorific value calculation formula creation system 100 creates a calorific value calculation formula of mixed gas will be described.

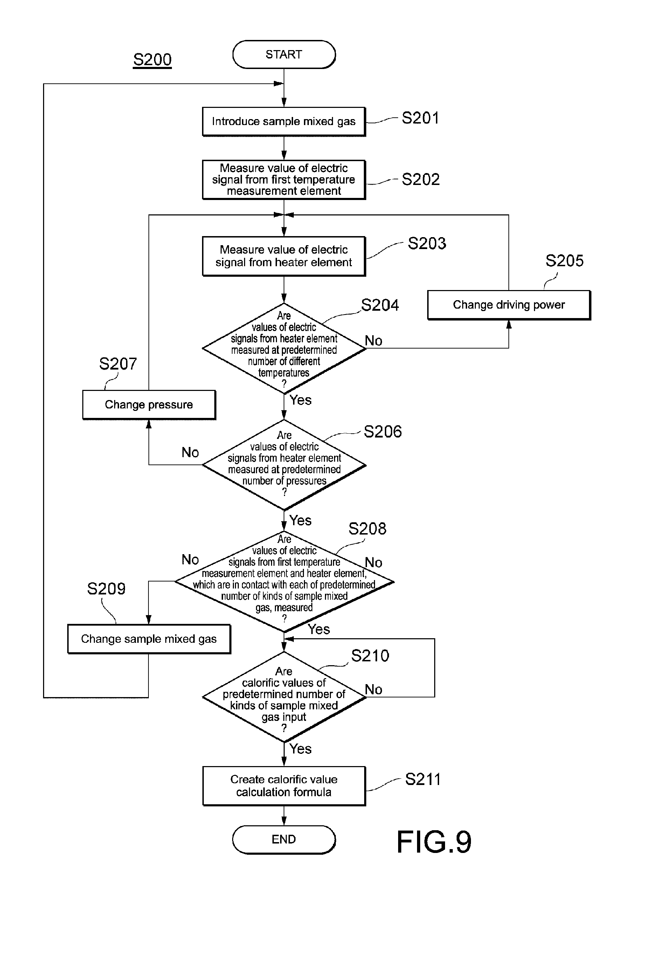

Fig. 9 is a flowchart illustrating an example of the behavior of the calorific value calculation formula creation system 100 of Fig. 1 when the calorific value calculation formula creation system 100 creates a calorific value calculation formula of mixed gas. For example, when the calorific value calculation formula creation system 100 of Fig. 1 is booted up, the controller 150 reads a program stored in a ROM or the like, and executes calorific value calculation formula creation processing S200 of Fig. 9.

First, the measurement module 151 of Fig. 1 introduces one of a plurality of kinds of mixed gas as samples (hereinafter arbitrarily referred to as sample mixed gas), whose calorific values are known, in the chamber 110 via the flow path 102 (S201).

In the example of Fig. 8, the measurement module 151 opens the valve of the first flow rate controller 32A and the valve of the pressure adjuster 94 while the valves of the second to fourth flow rate controllers 32B, 32C, and 32D are closed. As a result, the first sample mixed gas is injected into the chamber 110 through the flow paths 91 A, 92A, and 102. The gas in the chamber 110 is exhausted through the flow path 103, and is replaced by the first sample mixed gas.

Further, because the measurement module 151 opens the valve of the pressure adjuster 94 to thereby open the chamber 110, the pressure of the first sample mixed gas introduced in the chamber 110 reaches the atmospheric pressure.

Next, the measurement module 151 measures the value of the electric signal $S_I$ from the first temperature measurement element 62 of Fig. 2 and Fig. 3, and writes the measured value of the electric signal $S_I$ from the first temperature measurement element 62 (hereinafter arbitrarily referred to as measured value) in the storage 160. The storage 160 stores the measured value (S202). At this time, a weak voltage, which is not enough to generate self-heating, is applied to the first temperature measurement element 62. The measured value of the electric signal $S_I$ from the first temperature measurement element 62 depends on the temperature of sample mixed gas in contact with the first temperature measurement element 62.

In the example of Fig. 8, the measurement module 151 measures the value of the electric signal $S_I$ from the first temperature measurement element 62 in contact with the first sample mixed gas of the chamber 110. That is, the measurement module 151 measures the value of the electric signal $S_I$ from the first temperature measurement element 62 in contact with the first sample mixed gas having the atmospheric pressure.

Next, the measurement module 151 causes the driving circuit 130 to supply driving power to the heater element 61 of Fig. 2 and Fig. 3. As a result, the heater element 61 generates heat at a predetermined temperature. The measurement module 151 measures the value of the electric signal $S_H$ from the heater element 61, which generates heat at the predetermined temperature. The measurement module 151 writes the measured value of the electric signal from the heater element 61 (hereinafter arbitrarily referred to as measured value) in the storage 160, whereby the storage 160 stores the measured value (S203).

In the example of Fig. 8, the measurement module 151 measures the value of the electric signal $S_H$ from the heater element 61, which is in contact with the first sample mixed gas in the chamber 110. That is, the measurement module 151 measures the value of the electric signal $S_H$ from the heater element 61, which is in contact with the first sample mixed gas having the atmospheric pressure (i.e., first pressure) and generates heat at a predetermined temperature.

Note that the value of the electric signal $S_I$ from the first temperature measurement element 62 may be any one of the value of the resistance value $R_I$ of the first temperature measurement element 62, the value of the line current $I_I$ of the first temperature measurement element 62, the value of the voltage $V_I$ applied to the first temperature measurement element 62, and the value of the signal $AD_I$ output from the A/D converter 140 connected to the first temperature measurement element 62. Similarly, the value of the electric signal $S_H$ from the heater element 61 may be any one of the value of the resistance value $R_H$ of the heater element 61, the value of the line current $I_H$ of the heater element 61, the value of the voltage $V_H$ applied to the heater element 61, and the value of the signal $AD_H$ output from the A/D converter 140 connected to the heater element 61.

Next, the measurement module 151 determines if the measurement module 151 has measured values of electric signals $S_H$ from the heater element 61 at a predetermined number of different temperatures or not (S204). For example, the measurement module 151 determines if the measurement module 151 has measured values of electric signals $S_H$ at a predetermined number of different temperatures or not based on the number of the measured values of the electric signals $S_H$ from the heater element 61, which is stored in the storage 160.

The predetermined number of temperature depends on the number of the independent variables of the electric signals $S_H$ from the heater element 61 of a calorific value calculation formula to be created. Any number of different temperatures may be employed as long as the predetermined number is plural. In the example of Fig. 8, "5" is preset as the predetermined number.

If it is determined in S204 that the measurement module 151 has not measured the values of the electric signals $S_H$ from the heater element 61 at a predetermined number of different temperatures, the measurement module 151 changes the driving power supplied from the driving circuit 130 (S205), and executes Step S203 again. The measurement module 151 repeats Steps S203 to S205 until it measures the values of the electric signals $S_H$ from the heater element 61 at a predetermined number of different temperatures.

For example, let's say that the predetermined number of different temperatures is "5". First, in Step S203, if the driving circuit 130 supplies the driving power $P_{H1}$ and the heater element 61 generates heat at a temperature $TH_1$, the measurement module 151 measures the value of the electric signal $S_{H1}(T_{H1})$ from the heater element 61 when the heater element 61 generates heat at the temperature $T_{H1}$. Next, in Step S205, the driving power $P_{H1}$ supplied from the driving circuit 130 is changed to the driving power $P_{H2}$ larger than the driving power $P_{H1}$. Then, in Step S203, the driving power $P_{H2}$ is supplied to the heater element 61, and the heater element 61 generates heat at the temperature $T_{H2}$. Then the measurement module 151 measures the value of the electric signal $S_{H2}(T_{H2})$ from the heater element 61 when the heater element 61 generates heat at the temperature $T_{H2}$. Next, in Step S205, the driving power $P_{H2}$ supplied from the driving circuit 130 is changed to the driving power $P_{H3}$ larger than the driving power $P_{H2}$. Then, in Step S203, the driving power $P_{H3}$ is supplied to the heater element 61, and the heater element 61 generates heat at the temperature $T_{H3}$. Then the measurement module 151 measures the value of the electric signal $S_{H3}(T_{H3})$ from the heater element 61 when the heater element 61 generates heat at the temperature $T_{H3}$. Next, in Step S205, the driving power $P_{H3}$ supplied from the driving circuit 130 is changed to the driving power $P_{H4}$ larger than the driving power $P_{H3}$. Then, in Step S203, the driving power $P_{H4}$ is supplied to the heater element 61, and the heater element 61 generates heat at the temperature $T_{H4}$. Then the measurement module 151 measures the value of the electric signal $S_{H4}(T_{H4})$ from the heater element 61 when the heater element 61 generates heat at the temperature $T_{H4}$. Next, in Step S205, the driving power $P_{H4}$ supplied from the driving circuit 130 is changed to the driving power $P_{H5}$ larger than the driving power $P_{H4}$. Then, in Step S203, the driving power $P_{H5}$ is supplied to the heater element 61, and the heater element 61 generates heat at the temperature $T_{H5}$. Then the measurement module 151 measures the value of the electric signal $S_{H5}(T_{H5})$ from the heater element 61 when the heater element 61 generates heat at the temperature $T_{H5}$. As a result, the values of the electric signals $S_H$ from the heater element 61, which is in contact with the sample mixed gas having the first pressure and generates heat at a plurality of different temperatures, are measured.

Meanwhile, if it is determined in S204 that the measurement module 151 has measured the values of the electric signals $S_H$ from the heater element 61 at a predetermined number of different temperatures, then the measurement module 151 determines if the measurement module 151 has measured the values of the electric signals from the heater element 61 at a predetermined number of pressures or not (S206). For example, the measurement module 151 determines if the measurement module 151 has measured values of electric signals $S_H$ at a predetermined number of pressures or not based on the number of the measured values of the electric signals $S_H$ from the heater element 61, which is stored in the storage 160.

Any number of pressures may be employed as long as the predetermined number is plural. For example, "2" is preset as the predetermined number. In this case, the measurement module 151 determines if the number of the measured values of the electric signals $S_H$ from the heater element 61 stored in the storage 160 is, for example, "10" (=2x5 (predetermined number of pressures)x(predetermined number of different temperatures)) or not. Based on the result, the measurement module 151 determines if the measurement module 151 has measured the values of the electric signals from the heater element 61 at a predetermined number of pressures or not.

If it is determined in S206 that the measurement module 151 has not measured the values of the electric signals $S_H$ from the heater element 61 at a predetermined number of pressures, the measurement module 151 changes the pressure of the chamber 110 (S207), and executes Step S204 again. The measurement module 151 repeats Steps S204 to S207 until the measurement module 151 measures the values of the electric signals $S_H$ from the heater element 61 at a predetermined number of pressures.

In the example of Fig. 8, the measurement module 151 closes the valve of the pressure adjuster 94, whereby the pressure of the first sample mixed gas in the chamber 110 reaches a predetermined pressure adjusted by the first gas pressure adjuster 31 A, e.g., 20 [kPa]. As a result, the value of the electric signal $S_H$ from the heater element 61, which is in contact with the sample mixed gas having a second pressure different from the first pressure (i.e., atmospheric pressure), is measured.

Meanwhile, if it is determined in S206 that the measurement module 151 has measured the values of the electric signals $S_H$ from the heater element 61 at a predetermined number of pressures, then the measurement module 151 determines if the measurement module 151 has measured the values of the electric signals $S_I$ from the first temperature measurement element 62 and the values of the electric signals $S_H$ from the heater element 61, which are in contact with each of a predetermined number of kinds of sample mixed gas, or not (S208). For example, the measurement module 151 determines if the measurement module 151 has measured the values of the electric signals $S_I$ from the first temperature measurement element 62 and the values of the electric signals $S_H$ from the heater element 61, which are in contact with each of a predetermined number of kinds of sample mixed gas, based on the number of the measured values of the electric signals $S_I$ from the first temperature measurement element 62, which is stored in the storage 160.

Any number of sample mixed gases may be employed as long as the predetermined number is plural. For example, "4" is preset as the predetermined number.

If it is determined in S208 that the measurement module 151 has not measured the values of the electric signals $S_I$ from the first temperature measurement element 62 and the values of the electric signals $S_H$ from the heater element 61, which are in contact with each of a predetermined number of kinds of sample mixed gas, the measurement module 151 changes the sample mixed gas in the chamber 110 (S209), and executes Step S201 again. The measurement module 151 repeats Steps S201 to S209 until the measurement module 151 measures the values of the electric signals $S_I$ from the first temperature measurement element 62 and the values of the electric signals $S_H$ from the heater element 61, which are in contact with each of a predetermined number of kinds of sample mixed gas.

In the example of Fig. 8, first, when the first sample mixed gas is introduced in the chamber 110 in Step S201, in Step S209, the measurement module 151 opens the valve of the second flow rate controller 32B and the valve of the pressure adjuster 94 while the valves of the first, third, and fourth flow rate controllers 32A, 32C, and 32D are closed. Then, in Step S201, the second sample mixed gas is injected into the chamber 110 through the flow paths 91 B, 92B, and 102. The first sample mixed gas in the chamber 110 is exhausted through the flow path 103, and is replaced by the second sample mixed gas. Further, because the measurement module 151 opens the valve of the pressure adjuster 94 to thereby open the chamber 110, the pressure of the second sample mixed gas introduced in the chamber 110 reaches the atmospheric pressure. Next, when the second sample mixed gas is introduced in the chamber 110, in Step S209, the measurement module 151 opens the valve of the third flow rate controller 32C and the valve of the pressure adjuster 94 while the valves of the first, second, and fourth flow rate controllers 32A, 32B, and 32D are closed. Then, in Step S201, the third sample mixed gas is injected into the chamber 110 through the flow paths 91 C, 92C, and 102. The second sample mixed gas in the chamber 110 is exhausted through the flow path 103, and is replaced by the third sample mixed gas. Further, because the measurement module 151 opens the valve of the pressure adjuster 94 to thereby open the chamber 110, the pressure of the third sample mixed gas introduced in the chamber 110 reaches the atmospheric pressure. Next, when the third sample mixed gas is introduced in the chamber 110, in Step S209, the measurement module 151 opens the valve of the fourth flow rate controller 32D and the valve of the pressure adjuster 94 while the valves of the first to third flow rate controllers 32A, 32B, and 32C are closed. Then, in Step S201, the fourth sample mixed gas is injected into the chamber 110 through the flow paths 91 D, 92D, and 102. The third sample mixed gas in the chamber 110 is exhausted through the flow path 103, and is replaced by the fourth sample mixed gas. Further, because the measurement module 151 opens the valve of the pressure adjuster 94 to thereby open the chamber 110, the pressure of the fourth sample mixed gas introduced in the chamber 110 reaches the atmospheric pressure. As a result, the measurement module 151 measures the values of the electric signals $S_I$ from the first temperature measurement element 62 in contact with the first to fourth sample mixed gases, respectively, and the values of the electric signals $S_H$ from the heater element 61, which is in contact with the first to fourth sample mixed gases, respectively.

Meanwhile, if it is determined in S208 that the measurement module 151 has measured the values of the electric signals from the first temperature measurement element 62 and the heater element 61, which are in contact with each of a predetermined number of kinds of sample mixed gas, the measurement module 151 determines if the calorific values Q of a predetermined number of kinds of sample mixed gas have been input or not (S210). The measurement module 151 repeats Step S210 until the calorific values Q of a predetermined number of kinds of sample mixed gas are input.

A user inputs the calorific values Q of the sample mixed gases by using the input apparatus 170, for example. If the calorific values Q of the sample mixed gases are input, the controller 150 writes the input calorific values Q of the sample mixed gases in the storage 160, whereby the storage 160 stores the input calorific values Q.

In the example of Fig. 8, the known calorific value Q of the first sample mixed gas, the known calorific value Q of the second sample mixed gas, the known calorific value Q of the third sample mixed gas, and the known calorific value Q of the fourth sample mixed gas are input. In this case, the measurement module 151 determines that the calorific values Q of the plurality of kinds of sample mixed gas are input when the calorific values Q of all the first to fourth sample mixed gases are stored.

If it is determined in S210 that the calorific values Q of the plurality of kinds of sample mixed gas are input, the calculation formula creation module 152 reads the input known calorific values Q, the measured values of the electric signals $S_I$ from the first temperature measurement element 62, and the measured values of the electric signals $S_H$ from the heater element 61, from the storage 160. The calculation formula creation module 152 calculates and creates a calorific value calculation formula by multivariate analysis based on the calorific values Q of the plurality of kinds of sample mixed gas, the measured values of the electric signals $S_I$ from the first temperature measurement element 62, which is in contact with each of the plurality of kinds of sample mixed gas, the measured values of the electric signals $S_H$ from the heater element 61, which is in contact with each of the plurality of kinds of sample mixed gas having the first pressure and generates heat at a plurality of different temperatures, and the measured values of the electric signals $S_H$ from the heater element 61, which is in contact with each of the plurality of kinds of sample mixed gas having the second pressure different from the first pressure. The calculation formula creation module 152 writes the created calorific value calculation formula in the storage 160, whereby the storage 160 stores the created calorific value calculation formula (S211). Here, in the created calorific value calculation formula, the values of the electric signals $S_I$ from the first temperature measurement element 62 and the values of the electric signals $S_H$ from the heater element 61, which generates heat at the plurality of above-mentioned different temperatures, are independent variables, and the calorific value Q of measurement target mixed gas different from the plurality of kinds of sample mixed gas, whose calorific value is unknown, is a dependent variable.

The "multivariate analysis" includes a support vector regression and multiple regression analysis disclosed in A.J. Smola and B. Scholkopf, "A Tutorial on Support Vector Regression" (NeuroCOLT Technical Report (NC-TR-98-030), 1998) and Fuzzy Qualification Theory of Second Order disclosed in JP-A-5-141999.

After Step S210 is completed, the controller 150 completes the calorific value calculation formula creation processing S200.

In the example of this embodiment, in the created calorific value calculation formula, the values of the electric signals $S_I$ from the first temperature measurement element 62 and the values of the electric signals $S_H$ from the heater element 61 are independent variables. However, this embodiment is not limited to this example. As described with reference to the formula (28), if the temperature of mixed gas is constant, the electric signal $S_I$ from the first temperature measurement element 62 is a constant number. In this case, a calorific value calculation formula includes, as variables, only the electric signals $S_H$ from the heater element 61. That is, in S211, the calculation formula creation module 152 may read the input known calorific values Q and the measured values of the electric signals $S_H$ from the heater element 61 from the storage 160. The calculation formula creation module 152 may calculate and create a calorific value calculation formula by multivariate analysis based on the calorific values Q of the plurality of kinds of sample mixed gas, the measured values of the electric signals $S_H$ from the heater element 61, which is in contact with each of the plurality of kinds of sample mixed gas having the first pressure and generates heat at a plurality of different temperatures, and the measured values of the electric signals $S_H$ from the heater element 61, which is in contact with each of the plurality of kinds of sample mixed gas having the second pressure different from the first pressure. The calculation formula creation module 152 may write the created calorific value calculation formula in the storage 160, whereby the storage 160 stores the created calorific value calculation formula. In this case, in the created calorific value calculation formula, the values of the electric signals $S_H$ from the heater element 61, which generates heat at the plurality of above-mentioned different temperatures, are independent variables, and the calorific value Q of measurement target mixed gas different from the plurality of kinds of sample mixed gas, whose calorific value is unknown, is a dependent variable.

Here, the value of the electric signal $S_H$ from the heater element 61 depends on the pressure of mixed gas in contact with the heater element 61. In view of this, the inventors of the present invention have found that, if a calorific value calculation formula is created based on the values of the electric signals $S_H$ from the heater element 61, which is in contact with mixed gas having a plurality of pressures, an error of the calorific value Q of mixed gas calculated based on the calorific value calculation formula is small even if the pressure of mixed gas changes (i.e., the difference between the calculated calorific value Q and the true value is small). That is, as described above, the calculation formula creation module 152 creates a calorific value calculation formula based on the calorific values Q of the plurality of kinds of sample mixed gas, the measured values of the electric signals $S_H$ from the heater element 61, which is in contact with each of the plurality of kinds of sample mixed gas having the first pressure and generates heat at a plurality of different temperatures, and the measured values of the electric signals $S_H$ from the heater element 61, which is in contact with each of the plurality of kinds of sample mixed gas having the second pressure different from the first pressure. In the created calorific value calculation formula, the values of the electric signals $S_H$ from the heater element 61, which generates heat at the plurality of above-mentioned different temperatures, are independent variables, and the calorific value Q of measurement target mixed gas is a dependent variable. Because of this, it is possible to prevent calculation accuracy of the calorific value Q calculated based on the calorific value calculation formula from being decreased even if the pressure of mixed gas changes.

Further, the created calorific value calculation formula includes no pressure value of mixed gas as an independent variable. Because of this, it is possible to calculate, without measuring the pressure of mixed gas, a calorific value with a high degree of accuracy even if the pressure of mixed gas changes.

Further, the inventors of the present invention have found that, even if the created calorific value calculation formula includes the measured values of the electric signals $S_H$ from the heater element 61, which is in contact with each of sample mixed gases having the second pressure and generates heat at only one temperature, the accuracy of calculating a calorific value calculation formula is not decreased so much. In view of this, because the calorific value calculation formula includes the measured values of the electric signals $S_H$ from the heater element 61, which is in contact with each of a plurality of kinds of sample mixed gas having the second pressure and generates heat at one temperature out of a plurality of different temperatures, it is possible to reduce the number (number of times) of measuring the values of the electric signals $S_H$ from the heater element 61 when creating the calorific value calculation formula. As a result, it is possible to reduce the time to create a calorific value calculation formula significantly while the calculation accuracy is kept high even if the pressure of mixed gas changes.

(Calorific value measurement system and method of measuring a calorific value)

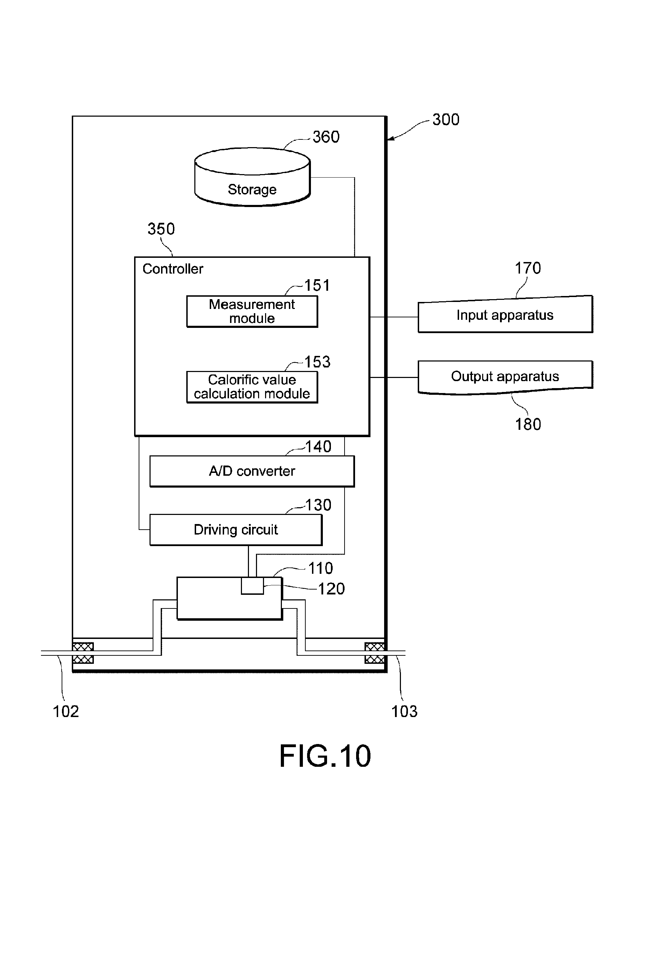

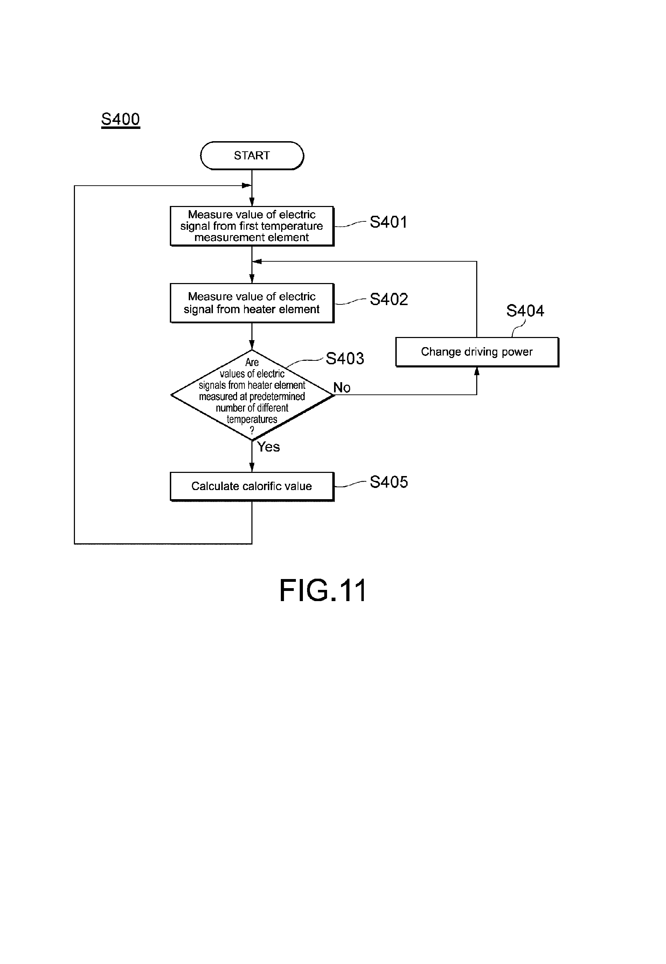

Fig. 10 and Fig. 11 show a calorific value measurement system and a method of measuring a calorific value according to an embodiment of the present invention. Note that, unless otherwise noted, configuration components same as the configuration components of the calorific value calculation formula creation system and the method of creating a calorific value calculation formula of the above-mentioned embodiment will be denoted by the same reference numerals, and description thereof will be omitted. Further, configuration components similar to the configuration components of the calorific value calculation formula creation system and the method of creating a calorific value calculation formula of the above-mentioned embodiment will be denoted by the similar reference numerals, and description thereof will be omitted. Further, not-shown configuration components, behaviors, and arrangements are similar to those of the calorific value calculation formula creation system and the method of creating a calorific value calculation formula of the above-mentioned embodiment.

Fig. 10 is a diagram schematically showing the configuration of the calorific value measurement system 300 of this embodiment. As shown in Fig. 10, the calorific value measurement system 300 is configured to measure a calorific value of mixed gas including a plurality of kinds of gas components, e.g., gas-phase liquified natural gas, etc. The calorific value measurement system 300 includes the above-mentioned chamber 110, the above-mentioned microchip 120, the above-mentioned driving circuit 130, the above-mentioned A/D converter 140, the controller 350, the storage 360, the above-mentioned input apparatus 170, and the above-mentioned output apparatus 180.

Similar to the above-mentioned controller 150 of the calorific value calculation formula creation system 100, the controller 350 is configured to control the respective blocks of the calorific value measurement system 300. Further, the controller 350 is configured to execute a method of measuring a calorific value (described later) according to the present invention. The controller 350 includes (i.e., functions as) the above-mentioned measurement module 151 and the calorific value calculation module 153.

The calorific value calculation module 153 is configured to calculate a calorific value of measurement target mixed gas, whose calorific value is unknown, based on the above-mentioned calorific value calculation formula.

Note that, similar to the above-mentioned controller 150 of the calorific value calculation formula creation system 100, the controller 350 includes, for example, a CPU, a GPU, memories such as a ROM, a RAM, and a buffer, various interfaces such as an input interface and an output interface, and a bus connecting them.

Similar to the above-mentioned storage 160 of the calorific value calculation formula creation system 100, the storage 360 is configured to store data. The controller 350 is connected to the storage 360 such that the controller 350 is capable of accessing the storage 360. The storage 360 is configured to store, for example, measured values of electric signals from the heater element 61 and the first temperature measurement element 62 of the microchip 120, a calorific value calculation formula created by the above-mentioned calorific value calculation formula creation system 100, and the like.

Note that the calorific value calculation formula created by the above-mentioned the calorific value calculation formula creation system 100 is read from the storage 160 of the calorific value calculation formula creation system 100 via, for example, a wired or wireless network or a removable medium (storage medium), and written in the storage 360, whereby the storage 360 stores the calorific value calculation formula.

Next, the behavior of the calorific value measurement system 300 when the calorific value measurement system 300 measures a calorific value Q of mixed gas will be described.

Fig. 11 is a flowchart illustrating an example of the behavior of the calorific value measurement system 300 of Fig. 10 when the calorific value measurement system 300 measures a calorific value Q of mixed gas. For example, in the calorific value measurement system 300 of Fig. 10, when measurement target mixed gas is introduced in the chamber 110 of Fig. 10, the controller 350 reads a program stored in a ROM or the like, and executes calorific value measurement processing S400 of Fig. 11.

First, the measurement module 151 of Fig. 10 measures a value of an electric signal $S_I$ from the first temperature measurement element 62 of Fig. 2 and Fig. 3. The measurement module 151 writes the measured value of the electric signal $S_I$ from the first temperature measurement element 62 in the storage 360. The storage 360 stores the measured value of the electric signal $S_I$ from the first temperature measurement element 62 (S401). At this time, a weak voltage, which is not enough to generate self-heating, is applied to the first temperature measurement element 62. The measured value of the electric signal $S_I$ from the first temperature measurement element 62 depends on the temperature of mixed gas in contact with the first temperature measurement element 62. In this manner, the value of the electric signal $S_I$ from the first temperature measurement element 62, which is in contact with measurement target mixed gas in the chamber 110, is measured.

Next, the measurement module 151 causes the driving circuit 130 to supply driving power to the heater element 61 of Fig. 2 and Fig. 3. As a result, the heater element 61 generates heat at a predetermined temperature. The measurement module 151 measures the value of the electric signal $S_H$ from the heater element 61, which generates heat at the predetermined temperature. The measurement module 151 writes the measured value of the electric signal $S_H$ from the heater element 61 in the storage 360, whereby the storage 360 stores the measured value (S402). In this manner, the value of the electric signal $S_H$ from the heater element 61, which is in contact with the measurement target mixed gas in the chamber 110, is measured.

The measurement module 151 determines if the measurement module 151 has measured the values of the electric signals $S_H$ from the heater element 61 at a predetermined number of different temperatures or not (S403). For example, the measurement module 151 determines if the measurement module 151 has measured values of electric signals $S_H$ at a predetermined number of different temperatures or not based on the number of the measured values of the electric signals $S_H$ from the heater element 61, which is stored in the storage 360.

The predetermined number depends on the number of the independent variables of the electric signal $S_H$ from the heater element 61 of the calorific value calculation formula stored in the storage 160. Any number of different temperatures may be employed as long as the predetermined number is plural. For example, "5" is preset as the predetermined number of temperature.

If it is determined in S403 that the measurement module 151 has not measured the values of the electric signals $S_H$ from the heater element 61 at a predetermined number of different temperatures, the measurement module 151 changes the power (watt) of the driving power supplied from the driving circuit 130 (S404), and executes Step S403 again. The measurement module 151 repeats Steps S402 to S404 until the measurement module 151 measures the values of the electric signals $S_H$ from the heater element 61 at a predetermined number of different temperatures. In this manner, the values of the electric signals $S_H$ from the heater element 61, which generates heat at a plurality of different temperatures, are measured.

For example, let's say that the predetermined number of different temperatures is "5". First, in Step S402, if the driving circuit 130 supplies the driving power $P_H$, and the heater element 61 generates heat at a temperature $TH_1$, the measurement module 151 measures the value of the electric signal $S_{H1}(T_{H1})$ from the heater element 61 when the heater element 61 generates heat at the temperature $T_{H1}$. Next, in Step S404, the driving power $P_{H1}$ supplied from the driving circuit 130 is changed to the driving power $P_{H2}$ larger than the driving power $P_{H1}$. Then, in Step S402, the driving power $P_{H2}$ is supplied to the heater element 61, and the heater element 61 generates heat at the temperature $T_{H2}$. Then the measurement module 151 measures the value of the electric signal $S_{H2}(T_{H2})$ from the heater element 61 when the heater element 61 generates heat at the temperature $T_{H2}$. Next, in Step S404, the driving power $P_{H2}$ supplied from the driving circuit 130 is changed to the driving power $P_{H3}$ larger than the driving power $P_{H2}$. Then, in Step S402, the driving power $P_{H3}$ is supplied to the heater element 61, and the heater element 61 generates heat at the temperature $T_{H3}$. Then the measurement module 151 measures the value of the electric signal $S_{H3}(T_{H3})$ from the heater element 61 when the heater element 61 generates heat at the temperature $T_{H3}$. Next, in Step S404, the driving power $P_{H3}$ supplied from the driving circuit 130 is changed to the driving power $P_{H4}$ larger than the driving power $P_{H3}$. Then, in Step S402, the driving power $P_{H4}$ is supplied to the heater element 61, and the heater element 61 generates heat at the temperature $T_{H4}$. Then the measurement module 151 measures the value of the electric signal $S_{H4}(T_{H4})$ from the heater element 61 when the heater element 61 generates heat at the temperature $T_{H4}$. Next, in Step S404, the driving power $P_{H4}$ supplied from the driving circuit 130 is changed to the driving power $P_{H5}$ larger than the driving power $P_{H4}$. Then, in Step S402, the driving power $P_{H5}$ is supplied to the heater element 61, and the heater element 61 generates heat at the temperature $T_{H5}$. Then the measurement module 151 measures the value of the electric signal $S_{H5}(T_{H5})$ from the heater element 61 when the heater element 61 generates heat at the temperature $T_{H5}$.

Meanwhile, if it is determined in S403 that the measurement module 151 has measured the values of the electric signals $S_H$ from the heater element 61 at a predetermined number of different temperatures, the calorific value calculation module 153 reads the measured value of the electric signal $S_I$ from the first temperature measurement element 62, the measured values of the electric signals $S_H$ from the heater element 61, which generates heat at a predetermined number of different temperatures, and the mixed gas calorific value calculation formula from the storage 360. The calorific value calculation module 153 substitutes the measured value of the electric signal $S_I$ from the first temperature measurement element 62 and the measured values of the electric signals $S_H$ from the heater element 61, which generates heat at a predetermined number of different temperatures, in the independent variables of the calorific value calculation formula, to thereby calculate the calorific value Q of mixed gas. The calorific value calculation module 153 outputs the calculated calorific value Q per unit volume to a display panel or the like of the output apparatus 180 (S405).

After Step S405, the controller 350 returns to Step S401, and repeats Steps S401 to S405 until the calorific value measurement system 300 stops, for example.

According to the example of this embodiment, the measured value of the electric signal $S_I$ from the first temperature measurement element 62 and the measured value of the electric signals $S_H$ from the heater element 61 are substituted in the independent variables of the calorific value calculation formula, whereby the calorific value Q of mixed gas is calculated. However, this embodiment is not limited to this example. As described in the calorific value calculation formula creation system 100 and the method of creating a calorific value calculation formula of the above-mentioned embodiment, the calorific value calculation formula may only include, as independent variables, the electric signals $S_H$ from the heater element 61. That is, in S403, the calorific value calculation module 153 may read the measured values of the electric signals $S_H$ from the heater element 61, which generates heat at a predetermined number of different temperatures, and the mixed gas calorific value calculation formula from the storage 360. The calorific value calculation module 153 may substitute the measured values of the electric signals $S_H$ from the heater element 61, which generates heat at a predetermined number of different temperatures, in the independent variables of the calorific value calculation formula, to thereby calculate the calorific value Q of mixed gas. The calorific value calculation module 153 may output the calculated calorific value Q per unit volume to a display panel or the like of the output apparatus 180.

Here, the calorific value calculation formula is created by the above-mentioned calorific value calculation formula creation system 100 by using the above-mentioned method of creating a calorific value calculation formula based on the calorific values Q of a plurality of kinds of sample mixed gas, the measured values of the electric signals $S_H$ from the heater element 61, which is in contact with each of the plurality of kinds of sample mixed gas having the first pressure and generates heat at a plurality of different temperatures, and the measured values of the electric signals $S_H$ from the heater element 61, which is in contact with each of the plurality of kinds of sample mixed gas having the second pressure different from the first pressure. Because of this, it is possible to prevent calculation accuracy of the calorific value Q calculated based on the calorific value calculation formula from being decreased even if the pressure of mixed gas changes.

Further, the calorific value calculation formula includes no pressure value of mixed gas as an independent variable. Because of this, it is possible to calculate, without measuring the pressure of mixed gas, a calorific value with a high degree of accuracy even if the pressure of mixed gas changes. Because of this, it is not necessary to provide a device (e.g., pressure sensor, etc.) for measuring pressure of mixed gas to the calorific value measurement system 300. The cost for manufacturing the calorific value measurement system 300 may be reduced.

Note that, in the following Example 1 and Example 2, the calorific value calculation formula created by the calorific value calculation formula creation system 100 by using the method of creating a calorific value calculation formula will be arbitrarily referred to as "the calorific value calculation formula of the present invention".


(Example 1)

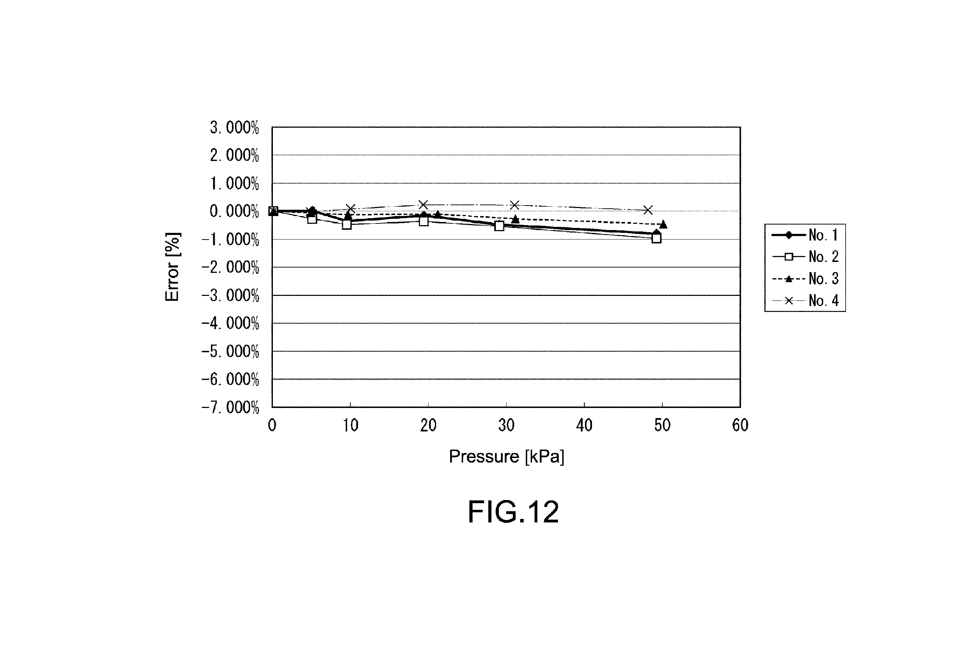

Each of Fig. 12 to Fig. 16 is a graph showing an example of relationship between pressure of mixed gas and an error (i.e., difference between calculated calorific value and true value) of a calorific value calculated based on the calorific value calculation formula of the present invention. Note that each graph of Fig. 12 to Fig. 16 shows a case where the pressures of four kinds of mixed gas (No. 1 to No. 4 of Fig. 12 to Fig. 16), whose calorific values Q are known, are changed. Each of the four kinds of sample mixed gas includes, as gas components, at least one of methane ($CH_4$), ethane ($C_2H_6$), propane ($C_3H_8$), and butane ($C_4H_{10}$) of different rates (percentages). For example, the sample mixed gas No. 1 only includes methane. Further, for example, the sample mixed gas No. 4 includes all of methane, ethane, propane, and butane. Meanwhile, in Fig. 12 to Fig. 16, the pressure (horizontal axis) is gauge pressure with reference to the atmospheric pressure. If the pressure of mixed gas is the same as the atmospheric pressure, the gauge pressure is 0. Further, in Fig. 12 to Fig. 16, the error (vertical axis) shows the difference between the calorific value Q calculated based on the calorific value calculation formula and the true value. The calorific value calculation formula of the present invention is created based on the measured values of the electric signals $S_H$ from the heater element 61, which is in contact with mixed gas having the first pressure (0 [kPa]) and generates heat at five different temperatures, and the measured values of the electric signals $S_H$ from the heater element 61, which is in contact with mixed gas having the second pressure (20 [kPa]) and generates heat at 23 [deg.]C.

If the temperature of measurement target mixed gas is 50 [deg.]C, as shown in Fig. 12, the error of a calorific value of each of all the mixed gases No. 1 to No. 4 having the pressure of 0 to 50 [kPa], which is calculated based on the calorific value calculation formula of the present invention, is equal to or less than ±1 [%]. Meanwhile, for comparison, a hypothetical calorific value calculation formula including no measured value of the electric signal $S_H$ from the heater element 61, which is in contact with the second mixed gas, (i.e., hypothetical calorific value calculation formula created only based on the measured values of the electric signals $S_H$ from the heater element 61, which is in contact with mixed gas of 0 [kPa] and generates heat at five different temperatures) is also prepared. The error of a calorific value calculated based on the hypothetical calorific value calculation formula is equal to or less than ±7 [%] under the same condition where the pressure of mixed gas is 0 to 50 [kPa].

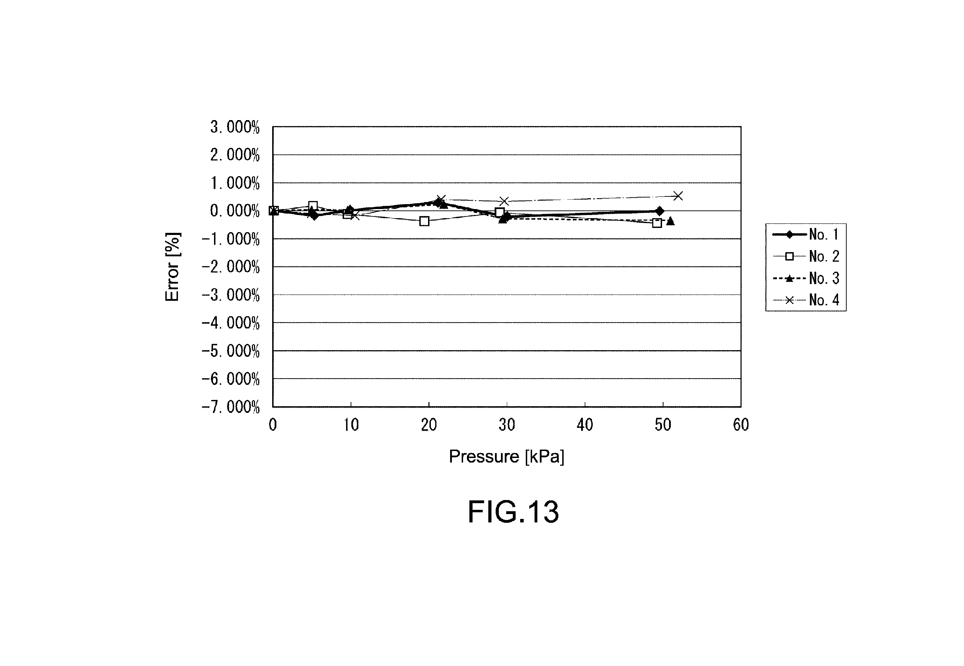

Further, if the temperature of measurement target mixed gas is 40 [deg.]C, as shown in Fig. 13, the error of a calorific value of each of all the mixed gases No. 1 to No. 4 having the pressure of 0 to 50 [kPa], which is calculated based on the calorific value calculation formula of the present invention, is equal to or less than about ±0.5 [%]. Meanwhile, the error of a calorific value calculated based on the hypothetical calorific value calculation formula is equal to or less than about ±6 [%] under the same condition where the pressure of mixed gas is 0 to 50 [kPa].

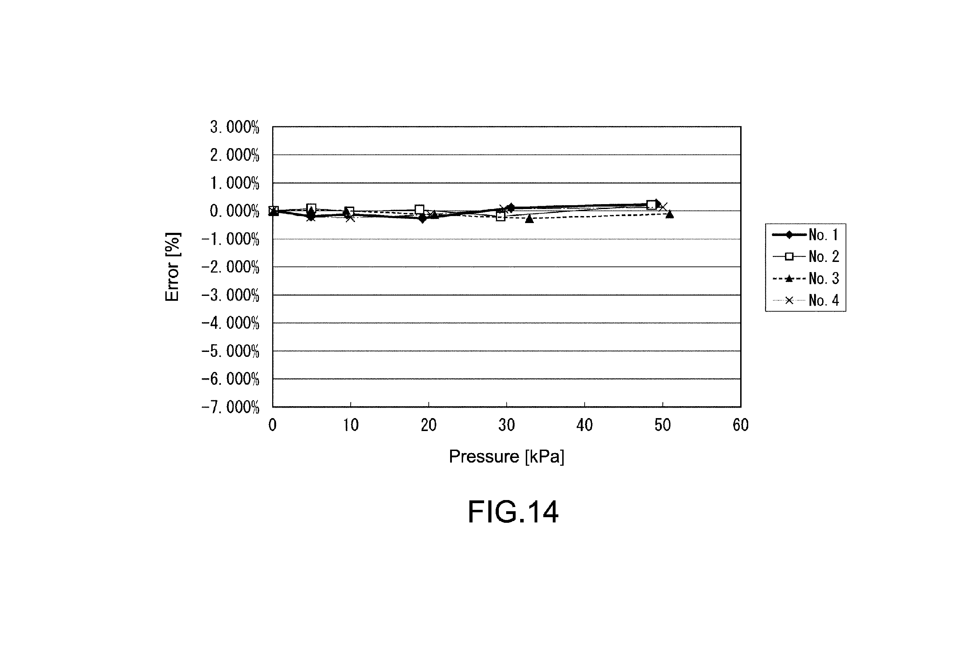

Further, if the temperature of measurement target mixed gas is 23 [deg.]C, as shown in Fig. 14, the error of a calorific value of each of all the mixed gases No. 1 to No. 4 having the pressure of 0 to 50 [kPa], which is calculated based on the calorific value calculation formula of the present invention, is equal to or less than about ±0.2 [%]. Meanwhile, the error of a calorific value calculated based on the hypothetical calorific value calculation formula is equal to or less than about ±6 [%] under the same condition where the pressure of mixed gas is 0 to 50 [kPa].

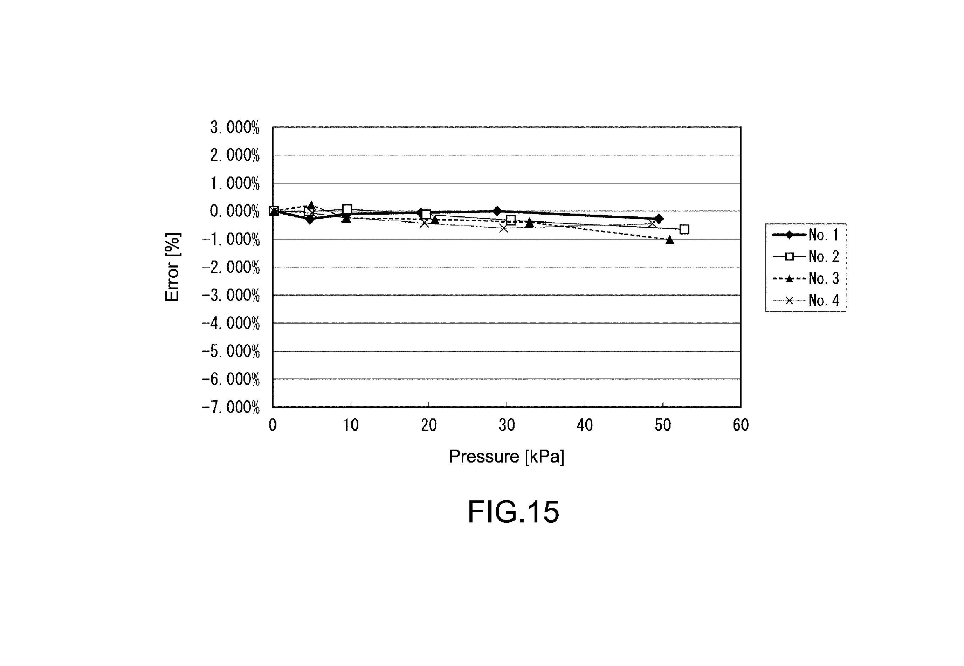

Further, if the temperature of measurement target mixed gas is 5 [deg.]C, as shown in Fig. 15, the error of a calorific value of each of all the mixed gases No. 1 to No. 4 having the pressure of 0 to 50 [kPa], which is calculated based on the calorific value calculation formula of the present invention, is equal to or less than about ±1 [%]. Meanwhile, the error of a calorific value calculated based on the hypothetical calorific value calculation formula is equal to or less than about ±6 [%] under the same condition where the pressure of mixed gas is 0 to 50 [kPa].

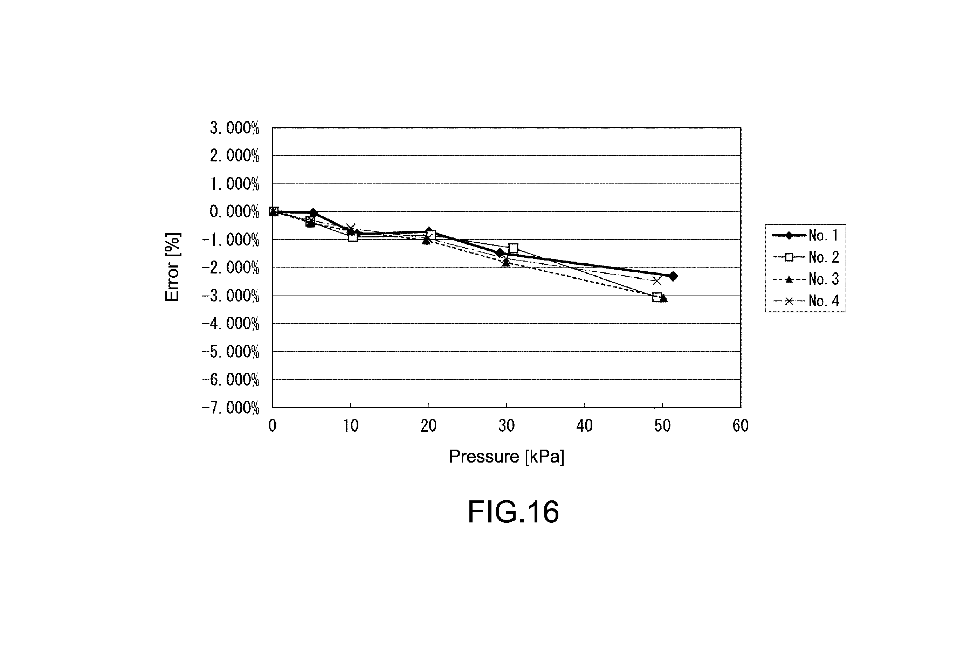

Further, if the temperature of measurement target mixed gas is -10 [deg.]C, as shown in Fig. 16, the error of a calorific value of each of all the mixed gases No. 1 to No. 4 having the pressure of 0 to 50 [kPa], which is calculated based on the calorific value calculation formula of the present invention, is equal to or less than about ±3 [%]. Meanwhile, the error of a calorific value calculated based on the hypothetical calorific value calculation formula is equal to or less than about ±6 [%] under the same condition where the pressure of mixed gas is 0 to 50 [kPa].

(Example 2)

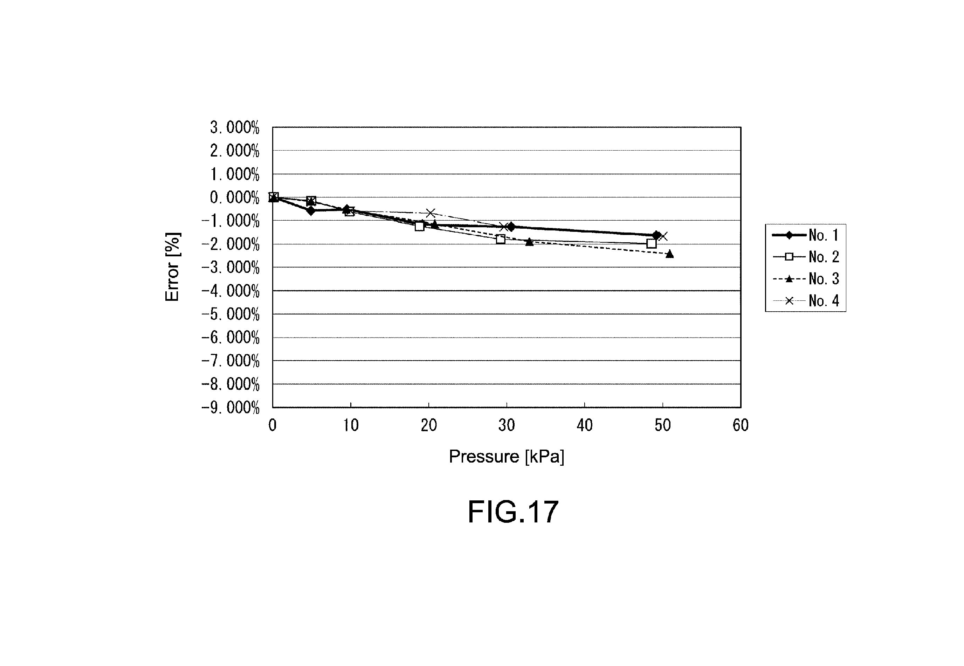

Fig. 17 is a graph showing an example of relationship between pressure of mixed gas and an error (i.e., difference between calculated calorific value and true value) of a calorific value calculated based on the calorific value calculation formula of the present invention. Note that, similar to the graphs of Fig. 12 to Fig. 16, the graph of Fig. 17 shows a case where the pressures of four kinds of mixed gas (No. 1 to No. 4 of Fig. 12 to Fig. 16), whose calorific values Q are known, are changed. The four kinds of sample mixed gas are the same as those shown in the graphs of Fig. 12 to Fig. 16. Further, similar to the graphs of Fig. 12 to Fig. 16, the pressure (horizontal axis) is gauge pressure in Fig. 17. Further, in Fig. 17, the error (vertical axis) shows the difference between the calorific value Q calculated based on the calorific value calculation formula and the true value. The calorific value calculation formula of the present invention is created based on the measured values of the electric signals $S_H$ from the heater element 61, which is in contact with mixed gas having the first pressure (0 [kPa]) and generates heat at four different temperatures, and the measured values of the electric signals $S_H$ from the heater element 61, which is in contact with mixed gas having the second pressure (20 [kPa]) and generates heat at 23 [deg.]C.

If the temperature of measurement target mixed gas is 23 [deg.]C, as shown in Fig. 17, the error of a calorific value of each of all the mixed gases No. 1 to No. 4 having the pressure of 0 to 50 [kPa], which is calculated based on the calorific value calculation formula of the present invention, is equal to or less than ±2.5 [%]. Meanwhile, for comparison, a hypothetical calorific value calculation formula including no measured value of the electric signal $S_H$ from the heater element 61, which is in contact with the second mixed gas, (i.e., hypothetical calorific value calculation formula created only based on the measured values of the electric signals $S_H$ from the heater element 61, which is in contact with mixed gas of 0 [kPa] and generates heat at four different temperatures) is also prepared. The error of a calorific value calculated based on the hypothetical calorific value calculation formula is equal to or less than ±9 [%] under the same condition where the pressure of mixed gas is 0 to 50 [kPa].

Note that, also in a case where the temperature of measurement target mixed gas is other than 23 [deg.]C, similarly, an error of a calorific value calculated based on the calorific value calculation formula of the present invention is smaller than an error of a calorific value calculated based on a hypothetical calorific value calculation formula (not shown).

Further, the calorific value calculation formula of the present invention is created based on the measured values of the electric signal $S_H$ from the heater element 61, which is in contact with mixed gas having the first pressure (0 [kPa]) and generates heat at three different temperatures, and the measured values of the electric signals $S_H$ from the heater element 61, which is in contact with mixed gas having the second pressure (20 [kPa]) and generates heat at 23 [deg.]C. In this case, also, an error of a calorific value calculated based on the calorific value calculation formula of the present invention is smaller than an error of a calorific value calculated based on a hypothetical calorific value calculation formula.

Note that the above-mentioned configuration components of the embodiments may be combined, or some configuration components may be interchanged. Further, the configuration of the present invention is not limited to the above-mentioned embodiments, and may be variously changed within the gist of the present invention.

**Claims**

1. A calorific value calculation formula creation system, comprising:

   a heater element (61) coming in contact with mixed gas;
   a measurement module (151) configured to measure a value of an electric signal from the heater element; and
   a calculation formula creation module (152) configured to create a calorific value calculation formula based

   on calorific values of the plurality of kinds of mixed gas,
   on measured values of electric signals from the heater element, the heater element being in contact with each of the plurality of kinds of mixed gas having a first pressure, the heater element generating heat at a plurality of different temperatures, and
   on measured values of electric signals from the heater element, the heater element being in contact with each of the plurality of kinds of mixed gas having a second pressure different from the first pressure, where the values of electric signals from the heater element, the heater element generating heat at the plurality of different temperatures, are independent variables, and
   a calorific value of the mixed gas is a dependent variable.

2. The calorific value calculation formula creation system according to claim 1, further comprising:

   a temperature measurement element (62, 63) in contact with the mixed gas, wherein
   the measurement module (151) is further configured to measure a value of an electric signal from the temperature measurement element (62, 63), and
   the calculation formula creation module (152) is configured to create the calorific value calculation formula further based on values of electric signals from the temperature measurement element, the temperature measurement element being in contact with each of the plurality of kinds of mixed gas, where the value of an electric signal from the temperature measurement element is further an independent variable.

3. The calorific value calculation formula creation system according to claim 1 or 2, wherein
   the measured values of electric signals from the heater element (61), the heater element being in contact with each of the plurality of kinds of mixed gas having the second pressure, are obtained when the heater element (61) generates heat at one temperature out of the plurality of different temperatures.

4. The calorific value calculation formula creation system according to any one of claims 1 to 3, wherein
   the calculation formula creation module (152) is configured to create the calorific value calculation formula by using support vector regression.

5. A method of creating a calorific value calculation formula, comprising:

   measuring a value of an electric signal from a heater element (61) coming in contact with mixed gas; and
   creating a calorific value calculation formula based

   on calorific values of the plurality of kinds of mixed gas,
   on measured values of electric signals from the heater element (61), the heater element being in contact with each of the plurality of kinds of mixed gas having a first pressure, the heater element (61) generating heat at a plurality of different temperatures, and
   on measured values of electric signals from the heater element (61), the heater element being in contact with each of the plurality of kinds of mixed gas having a second pressure different from the first pressure, where
the values of electric signals from the heater element (61), the heater element generating heat at the plurality of different temperatures, are independent variables, and
a calorific value of the mixed gas is a dependent variable.

6. The method of creating a calorific value calculation formula according to claim 5, further comprising:

measuring a value of an electric signal from a temperature measurement element (62, 63) in contact with the mixed gas, wherein
the calorific value calculation formula is created further based on values of electric signals from the temperature measurement element, the temperature measurement element (62, 63) being in contact with each of the plurality of kinds of mixed gas, where the value of an electric signal from the temperature measurement element (62, 63) is further an independent variable.

7. The method of creating a calorific value calculation formula according to claim 5 or 6, wherein the measured values of electric signals from the heater element (61), the heater element being in contact with each of the plurality of kinds of mixed gas having the second pressure, are obtained when the heater element generates heat at one temperature out of the plurality of different temperatures.

8. The method of creating a calorific value calculation formula according to any one of claims 5 to 7, wherein the calorific value calculation formula is created by using support vector regression.

9. A calorific value measurement system, comprising:

a heater element (61) coming in contact with mixed gas;
a measurement module (151) configured to measure a value of an electric signal from the heater element; and
a calorific value calculation module (153) configured

to substitute measured values of electric signals from the heater element, the heater element generating heat at a plurality of different temperatures, in independent variables of a calorific value calculation formula, where

the values of electric signals from the heater element, the heater element generating heat at the plurality of different temperatures, are the independent variables, and
a calorific value of the mixed gas is a dependent variable, and

to calculate the calorific value of the mixed gas, wherein

the calorific value calculation formula is created based

on calorific values of the plurality of kinds of mixed gas,
on measured values of electric signals from the heater element (61), the heater element (61) being in contact with each of the plurality of kinds of mixed gas having a first pressure, the heater element generating heat at a plurality of different temperatures, and
on measured values of electric signals from the heater element, the heater element being in contact with each of the plurality of kinds of mixed gas having a second pressure different from the first pressure.

10. The calorific value measurement system according to claim 9, further comprising:

a temperature measurement element (62, 63) in contact with the mixed gas, wherein
the measurement module (151) is further configured to measure a value of an electric signal from the temperature measurement element,
the calorific value calculation formula is created further based on values of electric signals from the temperature measurement element, the temperature measurement element being in contact with each of the plurality of kinds of mixed gas, where the value of an electric signal from the temperature measurement element is further an independent variable, and
the calorific value calculation module is configured

to further substitute the measured value of an electric signal from the temperature measurement element in the independent variable of the calorific value calculation formula, and

to calculate the calorific value of the mixed gas.

**11.** The calorific value measurement system according to claim 9 or 10, wherein
the calculation formula creation module is configured to create the calorific value calculation formula by using support vector regression.

**12.** A method of measuring a calorific value, comprising:

measuring a value of an electric signal from a heater element (61) coming in contact with mixed gas; and
substituting measured values of electric signals from the heater element (61), the heater element generating heat at a plurality of different temperatures, in independent variables of a calorific value calculation formula, and calculating a calorific value of the mixed gas, where

the values of electric signals from the heater element, the heater element generating heat at the plurality of different temperatures, are the independent variables, and
a calorific value of the mixed gas is a dependent variable, wherein

the calorific value calculation formula is created based

on calorific values of the plurality of kinds of mixed gas,
on measured values of electric signals from the heater element (61), the heater element (61) being in contact with each of the plurality of kinds of mixed gas having a first pressure, the heater element generating heat at a plurality of different temperatures, and
on measured values of electric signals from the heater element, the heater element being in contact with each of the plurality of kinds of mixed gas having a second pressure different from the first pressure.

**13.** The method of measuring a calorific value according to claim 12, further comprising:

measuring a value of an electric signal from a temperature measurement element in contact with the mixed gas, wherein
the calorific value calculation formula is created further based on values of electric signals from the temperature measurement element, the temperature measurement element being in contact with each of the plurality of kinds of mixed gas, where the value of an electric signal from the temperature measurement element is further an independent variable, and
the measured value of an electric signal from the temperature measurement element is further substituted in the independent variable of the calorific value calculation formula, and a calorific value of the mixed gas is calculated.

**14.** The method of measuring a calorific value according to claim 12 or 13, wherein
the calorific value calculation formula is created by using support vector regression.

**15.** The calorific value calculation formula creation system according to any one of claims 1 to 4, the method of creating a calorific value calculation formula according to any one of claims 5 to 8, the calorific value measurement system according to any one of claims 9 to 11, or the method of measuring a calorific value according to any one of claims 12 to 14, wherein
each of the plurality of kinds of mixed gas is liquified natural gas.

FIG.1

FIG.2

FIG.3

FIG.4

EP 2 833 130 A1

FIG.5

FIG.6

FIG.7

FIG.8

S200

```
                    ( START )
                        │
                        ▼
        ┌───────────────────────────┐
        │  Introduce sample mixed gas │──── S201
        └───────────────────────────┘
                        │
                        ▼
        ┌───────────────────────────┐
        │  Measure value of electric  │──── S202
        │ signal from first temperature│
        │   measurement element       │
        └───────────────────────────┘
                        │
                        ▼
        ┌───────────────────────────┐
        │  Measure value of electric  │──── S203
        │   signal from heater element │
        └───────────────────────────┘
                        │
                        ▼
                     ◇ S204                    ┌──────────────────────┐
            Are values of electric    ── No ──▶│  Change driving power  │ S205
          signals from heater element          └──────────────────────┘
          measured at predetermined
           number of different
              temperatures ?
                    │ Yes
                    ▼
                  ◇ S206
          Are values of electric
        signals from heater element   ── No ──▶┌──────────────────┐
        measured at predetermined              │  Change pressure  │ S207
         number of pressures ?                 └──────────────────┘
                    │ Yes
                    ▼
                  ◇ S208
          Are values of electric
   No ◀── signals from first temperature ── No
          measurement element and heater element,
        which are in contact with each of predetermined
          number of kinds of sample mixed
                gas, measured ?
                    │ Yes
   ┌──────────────────────┐
   │ Change sample mixed gas │ S209
   └──────────────────────┘
                    ▼
                  ◇ S210
          Are calorific values of
        predetermined number of    ── No
         kinds of sample mixed
              gas input ?
                    │ Yes
                    ▼
        ┌───────────────────────────┐
        │   Create calorific value    │──── S211
        │    calculation formula      │
        └───────────────────────────┘
                    │
                    ▼
                  ( END )
```

FIG.9

FIG.10

S400

```
           ┌─────────────┐
           │    START    │
           └─────────────┘
                  │
                  ▼
        ┌──────────────────────┐
        │  Measure value of    │
        │  electric signal     │──── S401
        │  from first          │
        │  temperature         │
        │  measurement element │
        └──────────────────────┘
                  │
                  ▼
        ┌──────────────────────┐           ┌──────────────────────┐
        │  Measure value of    │──── S402  │                 S404 │
        │  electric signal     │           │  Change driving power│
        │  from heater element │           └──────────────────────┘
        └──────────────────────┘
                  │              S403
                  ▼
              ╱───────╲
             ╱  Are    ╲
            ╱ values of  ╲
           ╱ electric     ╲  No
          ╱ signals from   ╲─────────────────┘
          ╲ heater element ╱
           ╲ measured at   ╱
            ╲ predetermined╱
             ╲ number of  ╱
              ╲ different ╱
               ╲ temp.  ╱
                ╲  ?   ╱
                 ╲───╱
                  │ Yes
                  ▼
        ┌──────────────────────┐
        │  Calculate calorific │──── S405
        │  value               │
        └──────────────────────┘
```

# FIG.11

FIG.12

FIG.13

FIG.14

FIG.15

FIG.16

FIG.17

**EUROPEAN SEARCH REPORT**

Application Number

EP 14 17 7437

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 372 359 A1 (YAMATAKE CORP [JP]) 5 October 2011 (2011-10-05) * paragraph [0047]; figures 1, 6 * * paragraphs [0049] - [0053]; figure 7 * * paragraph [0062] * | 1-15 | INV. G01N27/18 G01N33/22 |
| A | Claire Vallance: "Properties of Gases", , 2 February 2007 (2007-02-02), XP055139999, Retrieved from the Internet: URL:http://vallance.chem.ox.ac.uk/pdfs/Pro pertiesOfGasesLectureNotes.pdf [retrieved on 2014-09-12] * Section 5 * | 1-15 | |

TECHNICAL FIELDS SEARCHED (IPC)

G01N

The present search report has been drawn up for all claims

| Place of search | Date of completion of the search | Examiner |
|---|---|---|
| The Hague | 12 September 2014 | Marzocchi, Olaf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 14 17 7437

This annex lists the patent family members relating to the patent documents cited in the above-mentioned European search report.
The members are as contained in the European Patent Office EDP file on
The European Patent Office is in no way liable for these particulars which are merely given for the purpose of information.

12-09-2014

| Patent document cited in search report | Publication date | Patent family member(s) | Publication date |
|---|---|---|---|
| EP 2372359 A1 | 05-10-2011 | CN 102253078 A<br>EP 2372359 A1<br>JP 5335727 B2<br>JP 2011209008 A<br>KR 20110109853 A | 23-11-2011<br>05-10-2011<br>06-11-2013<br>20-10-2011<br>06-10-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010038285 A

- JP 5141999 A

**Non-patent literature cited in the description**

- **A.J. SMOLA ; B. SCHOLKOPF.** A Tutorial on Support Vector Regression. *NeuroCOLT Technical Report (NC-TR-98-030),* 1998